# EUROPEAN PATENT SPECIFICATION

(11) **EP 3 845 539 B1**
(45) Date of publication and mention of the grant of the patent: **16.11.2022**
(21) Application number: 20153435.1
(22) Date of filing: 23.01.2020
(51) Int. Cl.: C07D 498/04, C07D 513/04, A61P 35/00, A61K 31/424, A61K 31/429

(54) **IMIDAZOOXAZOLE DERIVATIVE HAVING ANTITUMOR EFFECT, AND PHARMACEUTICAL COMPOSITION INCLUDING SAME**
IMIDAZOOXAZOLDERIVAT MIT ANTITUMORWIRKUNG UND PHARMAZEUTISCHE ZUSAMMENSETZUNG DAMIT
DÉRIVÉ D'IMIDAZOOXAZOLE AYANT UN EFFET ANTITUMORAL ET COMPOSITION PHARMACEUTIQUE LE COMPRENANT

(30) Priority: 31.12.2019 US 201916731277
(43) Date of publication of application: 07.07.2021
(73) Proprietor: Korea Institute of Science and Technology, Seoul 02792 (KR)
(72) Inventor: MAHMOUD, Gamal El-din, 12622 Dokki-giza (EG); CHOI, Hong Seok, 61708 Gwangju (KR); YOO, Kyung Ho, 02792 Seoul (KR); HAN, Dong Keun, 02792 Seoul (KR); OH, Chang Hyun, 02792 Seoul (KR); MOHAMMED, Abdel-Maksoud, 12622 Dokki-giza (EG); MOHAMMED, El-Gamal I., 27272 Sharjah (AE); AMMAR, Usama M., 02792 Seoul (KR)
(74) Representative: Newcombe, Christopher David

(56) References cited:
- EP-A1- 3 483 166
- WO-A2-2004/110990
- JIN-HUN PARK ET AL: "Synthesis of New 6-(4-Fluorophenyl)-5-(2-substituted pyrimidin-4-yl)imidazo[2,1-b] thiazole Derivatives and their Antiproliferative Activity against Melanoma Cell Line", BULLETIN OF THE KOREAN CHEMICAL SOCIETY, vol. 31, no. 10, 20 October 2010 (2010-10-20), pages 2854-2860, XP055633644, KR ISSN: 0253-2964, DOI: 10.5012/bkcs.2010.31.10.2854
- ABDEL-MAKSOUD MOHAMMED S ET AL: "Design, synthesis, and anticancer activity of imidazo[2,1-b]oxazole-based RAF kinase inhibitors", BIOORGANIC CHEMISTRY, ACADEMIC PRESS INC., NEW YORK, NY, US, vol. 93, 10 October 2019 (2019-10-10), XP085903712, ISSN: 0045-2068, DOI: 10.1016/J.BIOORG.2019.103349 [retrieved on 2019-10-10]
- ABDEL-MAKSOUD MOHAMMED S ET AL: "Anticancer profile of newly synthesized BRAF inhibitors possess 5-(pyrimidin-4-yl)imidazo[2,1-b]thiazole scaffold", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, vol. 27, no. 10, 2 April 2019 (2019-04-02) , pages 2041-2051, XP085664277, ISSN: 0968-0896, DOI: 10.1016/J.BMC.2019.03.062
- MOHAMMED S. ABDEL-MAKSOUD ET AL: "Design, synthesis, in vitro antiproliferative evaluation, and kinase inhibitory effects of a new series of imidazo[2,1-b]thiazole derivatives", EUROPEAN JOURNAL OF MEDICINAL CHEMISTRY, vol. 95, 30 March 2015 (2015-03-30), pages 453-463, XP055569902, AMSTERDAM, NL ISSN: 0223-5234, DOI: 10.1016/j.ejmech.2015.03.065

## Description

### TECHNICAL FIELD

The present disclosure relates to an imidazooxazole derivative or a pharmaceutically acceptable salt thereof, a method of preparing the same, and a pharmaceutical composition for preventing or treating tumors including the same as an active ingredient.

### BACKGROUND ART

The mitogen-activated protein kinase (MAPK) signaling pathway (EGFR > Ras > Raf > Mek > Erk) is a signaling pathway involved in cell proliferation, which has been considered to be important in tumorigenesis in many studies since it was discovered in 1990.

There are three RAF kinase enzymes in humans: A-RAF, B-RAF, and C-RAF (Marais and Marshall Cancer Surv. 27: 101-125, 1996). Since mutations of B-RAF were found at a high frequency in human cancer, RAF proteins were recognized as a critical initiator and promoter of malignancy (Davies, H. et al. Nature 417: 949-954 (2002)).

B-RAF mutations have been identified in approximately 7 % of cancers, including 50 % to 70 % of melanomas, 35 % of ovarian cancers, 50 % of thyroid cancers, and 10 % of colorectal cancers (Tuveson, et al., Cancer Cell. 4:95-98 (2003); and Xing, Endocrine-Related Cancer: 12:245-262 (2005)). Approximately 90% of the mutations occur as a point mutation (V600E) in which a valine residue is changed to a glutamate residue in the kinase domain. This V600E is an important target for the development of cancer therapeutics. A V600E mutation confers an approximately 500-fold increase in kinase activity, resulting in hyperactivation of MEK and ERK and abnormal growth of tumor cells. Until now, about 40 B-RAF mutations have been found (mainly at the activation segment and the glycine-rich G-loop of the kinase catalytic domain). However, the occurrence of mutations other than V600E is fairly infrequent. In corectal cancer, about 10% of B-RAF mutations occur at the G-loop of the kinase domain (Rajagopalan et al., Nature 2002 418, 934).

Recent studies have found that knockdown of mutant B-RAF by siRNA in human melanoma cells inhibits both MEK and ERK, causing growth arrest of tumor cells and ultimately promoting apoptosis (Sharma, et al., Canfer Res. 65:2412-2421 (2005); and Wellbrock et al., Cancer Res. 64:2338-2342 (2004)). In addition, experimental results from short-hairpin RNA xenograft models targeting mutant B-RAF have shown that tumor regression resulting from B-RAF suppression is reversibly regulated (Hoeflich et al., Cancer Res. 66:999-1006 (2006). Taken together, *in-vivo* B-RAF signaling is strongly associated with tumorigenicity, confirming B-RAF as an important target for cancer therapeutics.

EP 3483166 discloses imidazooxazole derivative compounds for preventing and treating tumours. WO 2004/110990 discloses compounds capable of inhibiting p38 and methods for treating conditions associated with p38 activity or cytokine activity. Jin-Hun Park, et al: "Synthesis of new 6-(4-fluorophenyl)-5-(2-substituted pyrimidin-4-yl)imidazo[2,1-b] thiazole derivatives and their antiproliferative activity against melanoma cell line" Bulletin if the Korean Chemical Society, vol. 31, no. 10 (20 October 2010) discloses the synthesis of a series of pyrimidinyl-imidazo[2,1-b]thiazole derivatives and reviews their antiproliferative activity. Mohammed S. Abdel-Maksoud, et al "Designs, synthesis, and anticancer activity of imidazo[2,1-b]oxazole-based RAF kinase inhibitors" Bioorganic Chemistry, vol. 93 (10 October 2019) discloses a series of B-RAF kinase inhibitors having imidazo[2,1-b]oxazole scaffold and their anticancer activity. Mohammed S. Abdel-Maksoud, et al "Anticancer profile of newly synthesized BRAF inhibitors possess 5-(pyrimidin-4-yl)imidazo[2,1-b]thiazole scaffold" Bioorganic & Medicinal Chemistry, vol. 27, no. 10, pages 2041-2051 discloses a series of imidazo[2,1-b]thiazoles and their anticancer activities. Mohammed S. Abdel-Maksoud, et al "Designs, synthesis, in vitro antiproliferative evaluation, and kinase inhibitory effects of a new series of imidazo[2,1-b]thiazole derivatives" European Journal of Medicinal Chemistry, vol. 95, pages 453-463 (30 March 2015) discloses a series of 5,6-diarylimidazo[2,1-b]thiazole derivatives and their in vitro antiproliferative activities.

### DESCRIPTION OF EMBODIMENTS

### TECHNICAL PROBLEM

An aspect provides an imidazooxazole derivative compound or a solvate, stereoisomer, or pharmaceutically acceptable salt thereof.

Another aspect provides a pharmaceutical composition for use in preventing and treating tumors, the pharmaceutical composition including the imidazooxazole derivative compound or the solvate, stereoisomer, or pharmaceutically acceptable salt thereof as an active ingredient.

### SOLUTION TO PROBLEM

An aspect provides a compound represented by the following Chemical Formula P or a solvate, stereoisomer, or pharmaceutically acceptable salt thereof: 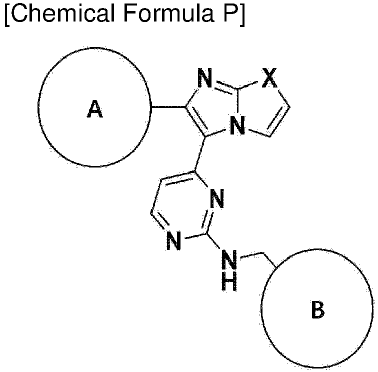 wherein, in Chemical Formula P,
X is O or S,
A is 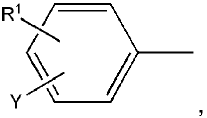 in which
R¹ is hydrogen, hydroxy, C₁-C₄ alkoxy, a halogen, nitro, or amino, and
Y is hydrogen, hydroxy, C₁-C₄ alkoxy, or a halogen;
B is 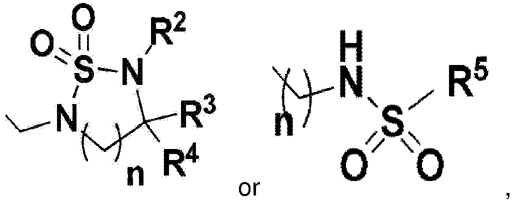 wherein
n = 1 or 2,
R², R³, and R⁴ are each independently hydrogen, *tert*-butyloxycarbonyl, or C₁-C₄ linear or branched alkyl, C₅-C₁₂ aryl, C₅-C₁₂ heteroaryl, or C₅-C₁₂ heterocycloalkyl unsubstituted or substituted with one or more substituents, and
R⁵ is M or M-Z, wherein M and Z are each independently C₅-C₁₂ aryl, C₅-C₁₂ heteroaryl, C₅-C₁₂ aryl C₁-C₄ alkyl, C₅-C₁₂ heteroaryl C₁-C₄ alkyl, C₅-C₁₂ cycloalkyl, or C₅-C₁₂ heterocycloalkyl unsubstituted or substituted with one or more substituents,
wherein the one or more substituents may be selected from the group consisting of halogen, C₁-C₄ linear or branched alkyl, C₁-C₄ alkoxy, trifluoromethyl, hydroxyl, amine, C₁-C₄ alkylamine, nitro, amide, C₁-C₄ alkylamide, urea, and acetyl.
In one embodiment, R¹ is hydrogen, hydroxy, C₁-C₄ alkoxy, a halogen, nitro, or amino;
Y is hydrogen, hydroxy, C₁-C₄ alkoxy, or a halogen;
R², R³, and R⁴ are each independently hydrogen or C₁-C₄ linear or branched alkyl; and
R⁵ is M or M-Z, wherein M and Z are each independently C₅-C₁₂ aryl, C₅-C₁₂ heteroaryl, C₅-C₁₂ cycloalkyl, or C₅-C₁₂ heterocycloalkyl unsubstituted or substituted with one or more substituents,
wherein the one or more substituents may be selected from the group consisting of a halogen, C₁-C₄ linear or branched alkyl, C₁-C₄ alkoxy, trifluoromethyl, and hydroxyl, amine, C₁-C₄ alkylamine, nitro, amide, C₁-C₄ alkylamide, urea, and acetyl, wherein the compound represented by Chemical Formula P is selected from the group consisting of:
   4-fluoro-N-(3-(4-(6-(4-fluoro-3-methoxyphenyl)imidazo[2,1-b]thiazol-5-yl) pyrimidin-2-ylamino)propyl)benzenesulfonamide;
   4-chloro-N-(3-(4-(6-(4-fluoro-3-methoxyphenyl)imidazo[2,1-b]thiazol-5-yl) pyrimidin-2-ylamino)propyl)benzenesulfonamide;
   4-fluoro-N-(2-(4-(6-(4-fluoro-3-hydroxyphenyl)imidazo[2,1-b]thiazol-5-yl) pyrimidin-2-ylamino)ethyl)benzenesulfonamide;
   4-chloro-N-(2-(4-(6-(4-fluoro-3-hydroxyphenyl)imidazo[2,1-b]thiazol-5-yl) pyrimidin-2-ylamino)ethyl)benzenesulfonamide;
   N-(2-(4-(6-(4-fluoro-3-hydroxyphenyl)imidazo[2,1-b]thiazol-5-yl)pyrimidin-2-yl amino)ethyl)benzenesulfonamide;
   4-chloro-N-(3-(4-(6-(4-fluoro-3-hydroxyphenyl)imidazo[2,1-b]thiazol-5-yl) pyrimidin-2-ylamino)propyl)benzenesulfonamide;
   N-(3-(4-(6-(4-fluoro-3-hydroxyphenyl)imidazo[2,1-b]thiazol-5-yl)pyrimidin-2-yl amino)propyl)-4-methoxybenzenesulfonamide;
   N-(3-(4-(6-(3-amino-4-fluorophenyl)imidazo[2,1-b]thiazol-5-yl)pyrimidin-2-yl amino)propyl)-4-fluorobenzenesulfonamide;
   4-chloro-N-(3-(4-(6-(4-fluoro-3-methoxyphenyl)imidazo[2,1-b]oxazol-5-yl) pyrimidin-2-ylamino)propyl)benzenesulfonamide;
   4-fluoro-N-(2-(4-(6-(4-fluoro-3-hydroxyphenyl)imidazo[2,1-b]oxazol-5-yl) pyrimidin-2-ylamino)ethyl)benzenesulfonamide;
   4-chloro-N-(2-(4-(6-(4-fluoro-3-hydroxyphenyl)imidazo[2,1-b]oxazol-5-yl) pyrimidin-2-ylamino)ethyl)benzenesulfonamide;
   4-chloro-N-(3-(4-(6-(4-fluoro-3-hydroxyphenyl)imidazo[2,1-b]oxazol-5-yl) pyrimidin-2-ylamino)propyl)benzenesulfonamide;
   N-(3-(4-(6-(4-fluoro-3-hydroxyphenyl)imidazo[2,1-b]oxazol-5-yl)pyrimidin-2-yl amino)propyl)-4-methoxybenzenesulfonamide; and
   N-(3-(4-(6-(4-fluoro-3-hydroxyphenyl)imidazo[2,1-b]oxazol-5-yl)pyrimidin-2-yl amino)propyl)benzenesulfonamide.

The 'halogen' may be F, Cl, Br, or I.

The 'alkoxy' may be methoxy, ethoxy, n-propoxy, isopropoxy, n-butoxy, isobutoxy, or t-butoxy.

The 'alkyl' refers to a linear or branched aliphatic hydrocarbon group having a particular number of carbon atoms, and may be methyl, ethyl, n-propyl, isopropyl, butyl, isobutyl, or t-butyl.

The 'aryl' may be phenyl, naphthyl, anthracenyl, indanyl, or biphenyl.

The 'heteroaryl' or 'heterocycloalkyl' may be azetidinyl, acridinyl, benzodioxolyl, benzodioxanyl, benzofuranyl, carbazolyl, cinnolinyl, dioxolanyl, pyridyl, pteridinyl, purinyl, quinazolinyl, quinoxalinyl, quinolinyl, isoquinolinyl, tetrazolyl, imidazolyl, tetrahydroisoquinolinyl, pyrrolyl, piperonyl, pyrazinyl, pyrimidinyl, pyridazinyl, pyrazolyl, oxazolyl, oxazolinyl, triazolyl, indanyl, isoxazolyl, isoxazolidinyl, decahydroisoquinolyl, benzimidazolyl, indazolyl, phenylpiperidinyl, furyl, tetrahydrofuryl, tetrahydropyranyl, piperazinyl, homopiperazinyl, piperidyl, piperidopiperidyl, morpholinyl, thiomorpholinyl, piperidinyl, 2-oxopiperazinyl, 2-oxopiperidinyl, pyrrolidinyl, 2-oxopyrrolidinyl, or oxazolidinyl.

The 'cycloalkyl' may be selected from cyclopropyl, cyclobutyl, cyclopentyl, cyclohexyl, cyclooctyl, cycloheptyl, perhydronaphthyl, adamantyl, cross-linked cyclic groups, and spirobicyclic groups.

An aspect provides a method of preparing the compound represented by Chemical Formula P or the solvate, stereoisomer, or pharmaceutically acceptable salt thereof, the method including:
cyclizing a compound of Chemical Formula 1 and a compound of Chemical Formula 2 to prepare a compound of Chemical Formula 3;
reacting the compound of Chemical Formula 3 with 4-chloro-2-(methylthio)pyrimidine to prepare a compound of Chemical Formula 4;
oxidizing the compound of Chemical Formula 4 to prepare a compound of Chemical Formula 5; and
reacting the compound of Chemical Formula 5 with an N-aminoethylcyclic sulfonamide derivative or an N-(2-aminoethyl)benzenesulfonamide derivative in the presence of a base to prepare the compound of Chemical Formula P.

Specifically, the preparation method may be represented by the following Reaction Scheme 1:

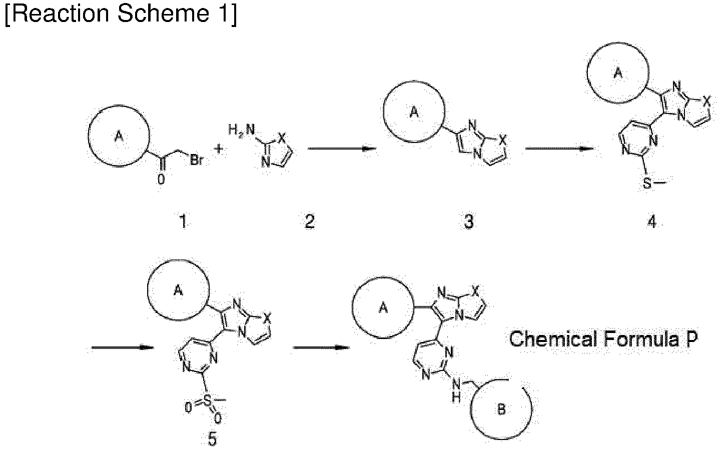

In Reaction Scheme 1, A, B, and X are the same as defined above.

In a specific embodiment, preparation of the compound of Chemical Formula 4 by reacting the compound of Chemical Formula 3 with 4-chloro-2-(methylthio)pyrimidine may be performed under Heck reaction conditions in the presence of Pd(OAc)₂ as a catalyst. Each procedure may further include purification as needed. In the procedure 4, the base may be diisopropylethylamine (DIPEA).

Another aspect provides a pharmaceutical composition for use in preventing and treating tumors, the pharmaceutical composition including the compound or the solvate, stereoisomer, or pharmaceutically acceptable salt thereof as an active ingredient.

The 'pharmaceutically acceptable salt' is any organic or inorganic acid addition salt of the compound of Chemical Formula P which is at such a concentration that is relatively nontoxic to a patient and has a harmless effective action, and adverse effects from the salt do not counteract benefits of the compound of Chemical Formula P. These salts may use an inorganic acid or an organic acid as a free acid. As an inorganic acid, hydrochloric acid, bromic acid, nitric acid, sulfuric acid, perchloric acid, phosphoric acid, etc. may be used. As an organic acid, citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicilic acid, citric acid, benzoic acid, malonic acid, etc. may be used. These salts may include alkali metal salts (sodium salt, potassium salt, etc.) and alkali earth metal salts (calcium salt, magnesium salt, etc.). For example, the acid addition salt may include acetate, aspartate, benzoate, besylate, bicarbonate/carbonate, bisulfate/sulfate, borate, camcilate, citrate, edisylate, ethylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, methylate, methylsulfate, naphthalate, 2-naphthylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, zinc salt, etc. Among them, hydrochloride or trifluoroacetate may be used.

In a specific embodiment, the compound or the solvate, stereoisomer, or pharmaceutically acceptable salt thereof may inhibit protein kinase to inhibit proliferation of tumor cells. The protein kinase is involved in proliferation of tumor cells, and may be one or more selected from the group consisting of V600E Rapidly Accelerated Fibrosarcoma (RAF), B-RAF, C-RAF, Mitogen-activated protein kinase 14 (MAPK14), Fms-like tyrosine kinase 3 (FLT3), and Glycogen synthase kinase 3 beta (GSK3β).

In another specific embodiment, the tumors may be lung cancer, liver cancer, esophageal cancer, stomach cancer, colorectal cancer, small intestine cancer, pancreatic cancer, melanoma, breast cancer, oral cancer, bone cancer, brain tumor, thyroid cancer, parathyroid cancer, renal cancer, cervical cancer, sarcoma, prostate cancer, urethral cancer, bladder cancer, testicular cancer, leukemia, multiple myeloma, myelodysplastic syndrome, hematological malignancy such as or glioblastoma, lymphoma such as Hodgkin's disease and non-Hodgkin lymphoma, Behcet's disease, skin cancer, psoriasis, and fibroadenoma.

The subject may be a mammal, and the mammal may be a human. An administration dose of the compound of the present disclosure which is effective for a human body may vary depending on a patient' age, body weight, sex, administration mode, health conditions, and severity of disease.

The administration may be performed by various formulations for oral administration or parenteral administration such as intravenous, intraperitoneal, intradermal, subcutaneous, epithelial, or intramuscular administration. The formulations may be prepared using a commonly used diluent or excipient such as a filler, an extender, a binder, a wetting agent, a disintegrant, a surfactant, etc.

Solid formulations for oral administration may include tablets, pills, powders, granules, capsules, troches, etc., and such a solid formulation is prepared by mixing one or more compounds of the present disclosure with at least one excipient, for example, starches, calcium carbonate, sucrose, lactose, gelatin, etc. Also, lubricants such as magnesium stearate and talc may be used in addition to simple excipients. Liquid formulations for oral administration may include suspensions, solutions for internal use, emulsions, syrups, etc. Various excipients, for example, wetting agents, sweetening agents, fragrances, preservatives, etc. may be included in addition to water and liquid paraffin which are simple diluents commonly employed.

Formulations for parenteral administration may include sterilized aqueous solutions, non-aqueous solutions, suspensions, emulsions, lyophilized formulations, suppositories, etc. Examples of non-aqueous solutions or suspensions may include propylene glycol, polyethylene glycol, vegetable oil such as olive oil, an injectable ester such as ethyl oleate, etc. Examples of suppository bases may include Witepsol, macrogol, Tween 61, cacao butter, laurin fat, glycerol, gelatin, etc.

### BRIEF DESCRIPTION OF DRAWINGS

FIG. 1 shows tumor size according to the number of days after transplantation, which represents anti-cancer effects after administration of compound 2II to A375P cell line xenograft models (G1 indicates an excipient control group, G2 indicates a group administered with 20 mg/kg/day of a test material, and G3 indicates a group administered with 50 mg/kg/day of a test material).
FIG. 2 shows graphs of inhibitory activity of compounds 37III and 42III against proliferation of the A375P melanoma cell line.

### MODE OF DISCLOSURE

Hereinafter, the present disclosure will be described in more details with reference to Examples. However, these Examples are for illustrative purposes only, and the scope of the present disclosure is not intended to be limited thereto. Only compounds according to claim 1 (compounds (37III), (38III), (42III), (43III), (46III), (48III), (50III), (52III), (60IV), (64IV), (65IV), (70IV), (72IV) and (73IV)) are examples of the invention. All other compounds are to be understood for reference.

### Preparation Method 1

Compounds of Chemical Formulae I to IV were prepared according to the following procedures, as shown in Reaction Schemes 2 to 5.

A method of synthesize an imidazooxazole compound is the same as in the following Reaction Scheme 2, and for synthesis of the compounds of Chemical Formulae I to IV, it is important to synthesize a compound 5 which is a main intermediate.

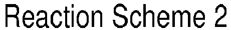

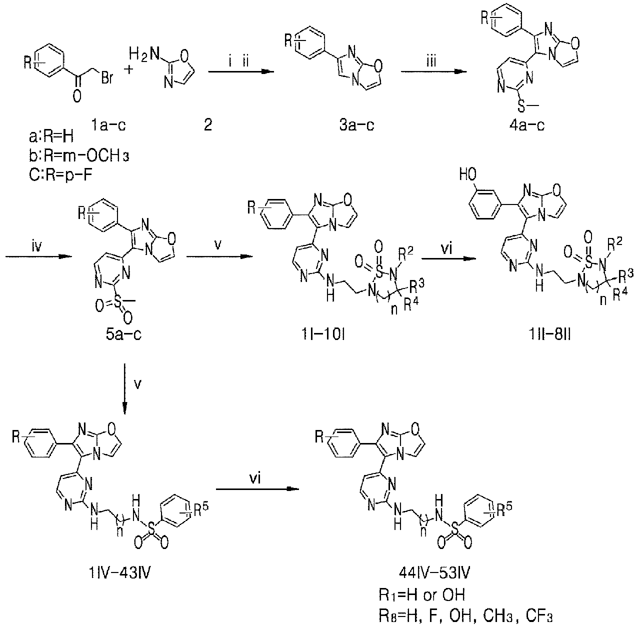

Reagents and Conditions: i) CH₃CN, THF, room temperature, 24 hours; ii) TiCl₄, toluene, reflux; iii) 4-chloro-2-(methylthio)pyrimidine, palladium acetate, triphenylphosphine, cesium carbonate, DMF, 80 °C, 16 hours; iv) oxone, methanol, H₂O; v) N-(2-aminoethyl)benzene sulfonamide or N-aminoethyl cyclicsulfonamide, DIPEA, DMSO, 80 °C; vi) BBr₃, dichloromethane.

Cyclization was carried out by reacting 2-aminooxazole (2) with α-bromo-4-fluoroacetophenone in ethanol under reflux for 16 hours, and crystallization was carried out using ammonia to synthesize a compound 3. The compound 3 thus synthesized was reacted with 4-chloro-2-(methylthio) pyrimidine in the presence of Pd(OAc)₂ catalyst under Heck reaction conditions at 80 °C for 36 hours. After the reaction was completed, column chromatography was used to obtain a methylthiopyrimidinyl compound 4. The compound 4 thus synthesized was oxidized using oxone at room temperature overnight, and purified by a column to synthesize a compound 5 which is a key intermediate. Under diisopropylethylamine, N-aminoethyl cyclic sulfonamide or N-(2-aminoethyl)benzenesulfonamide was dissolved in a DMF solvent, and stirred at 80 °C for 5 hours to 10 hours to obtain compounds I, II, and IV. Among these compounds, a methoxy compound was dissolved in methylene chloride, and cooled at -78 °C. Then, BBr₃ was slowly added dropwise, and stirred for 30 minutes. The temperature was raised to room temperature, and demethylation was carried out for 12 hours to synthesize a target compound having a hydroxyl group.

### Preparation Method 2

A method of synthesizing an imidazothiazole compound is the same as in the following Reaction Scheme 3, and the method was carried out in the same manner as in Reaction Scheme 2, except that 2-aminothiazole(2) was used.

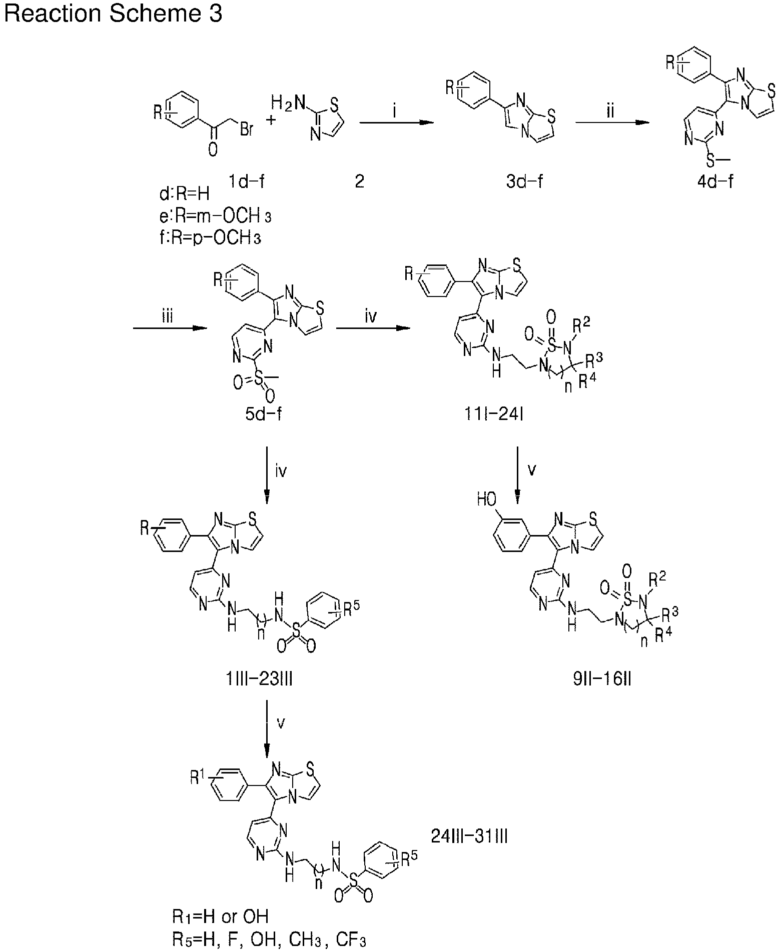

Reagents and Conditions: i) ethanol, reflux; overnight, ii) 4-chloro-2-(methylthio)pyrimidine, palladium acetate, triphenylphosphine, cesium carbonate, DMF, 80 °C, 16 hours; iii) oxone, methanol, H₂O; iv) N-(2-aminoethyl)benzenesulfonamide or N-aminoethyl cyclic sulfonamide, DIPEA, DMSO, 80 °C; v) BBr₃, dichloromethane.

### Preparation Method 3

Compounds of Chemical Formulae I to IV were prepared according to the following procedures, as shown in Reaction Schemes 4 and 5.

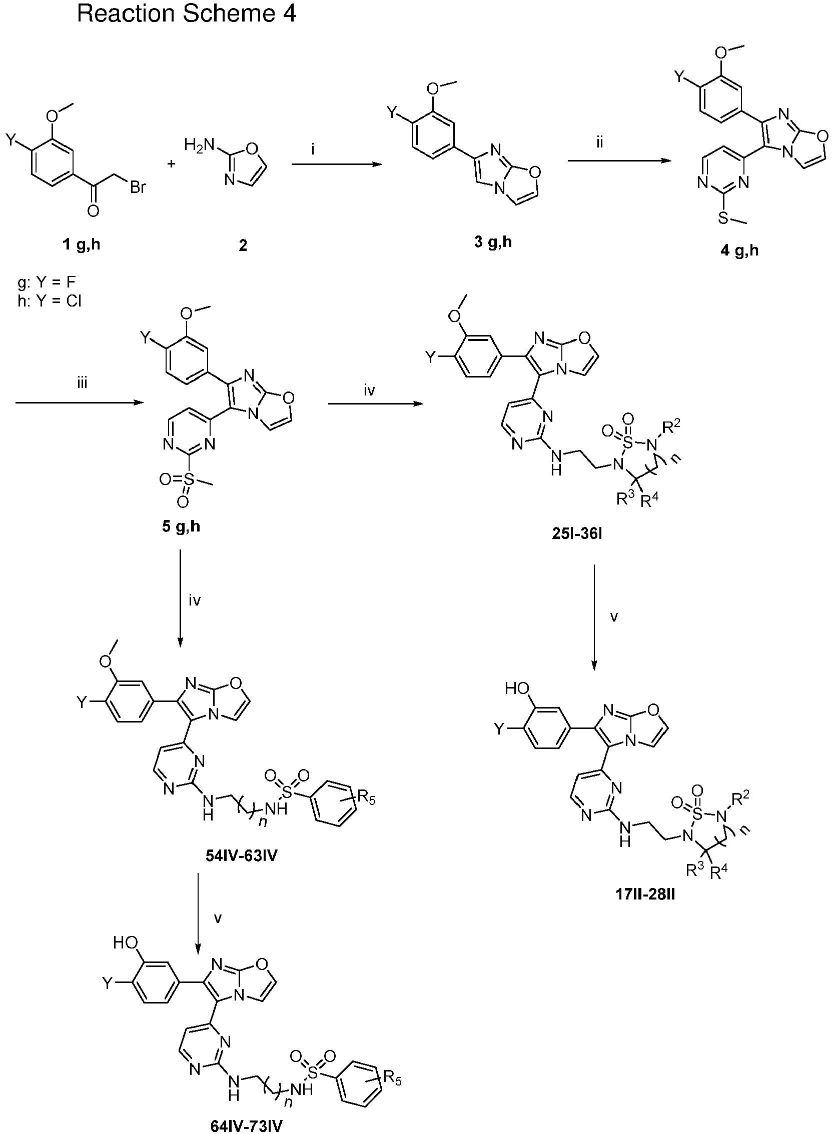

Reagents and and conditions: i) Ethanol, reflux; over night ii) 4-chloro-2-(methylthio) pyrimidine, palladium acetate, triphenylphosphine, cesium carbonate, DMF, 80 °C, 16h; iii) Oxone, Methanol, H₂O; iv) N-(2-Aminoethyl)benzenesulfonamide or N-Aminoethyl cylic sulfonamide, DIPEA, DMSO, 80°C; v) BBr₃, Dichloromethane.

### Preparation Method 4

A method of synthesizing an imidazothiazole compound is the same as in the following Reaction Scheme 5, and the method was carried out in the same manner as in Reaction Scheme 4, except that 2-aminothiazole(2) was used.

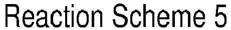

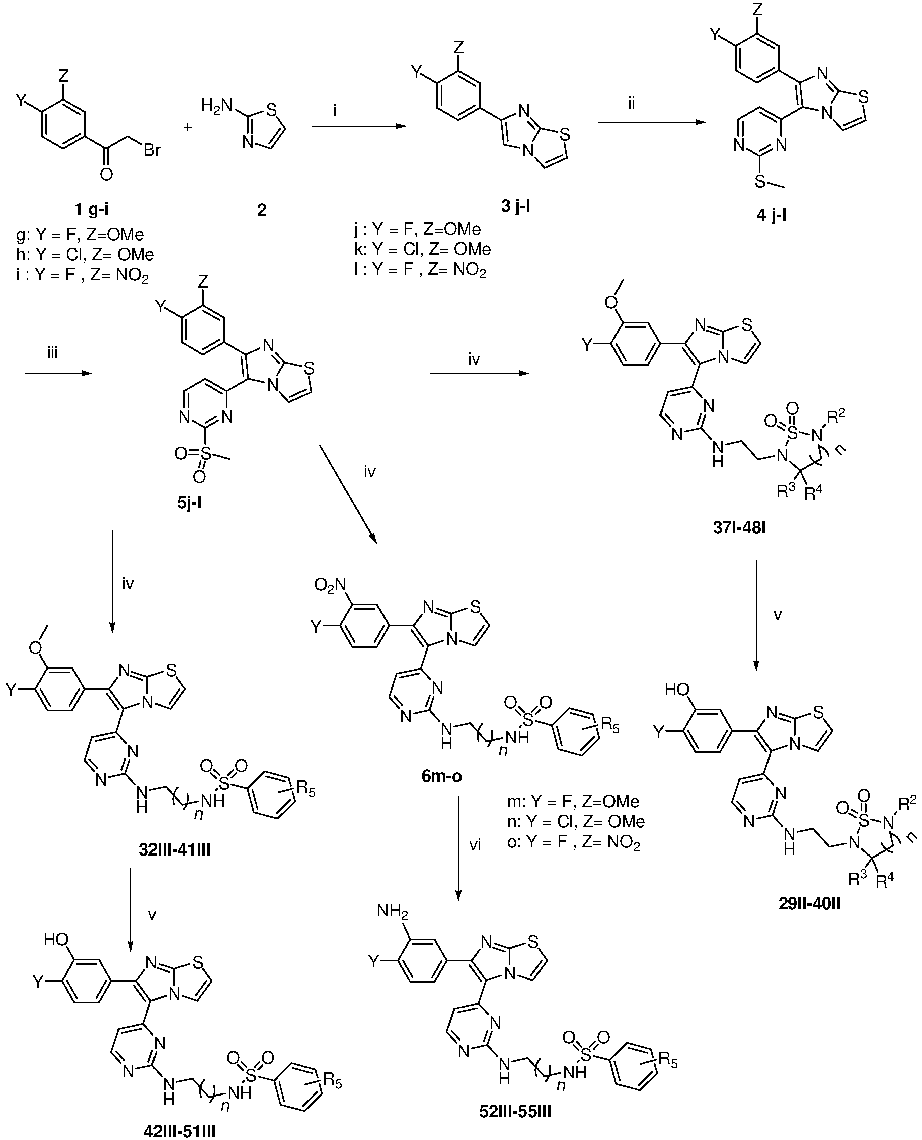

Reagents and conditions: i) Ethanol, reflux; over night ii) 4-chloro-2-(methylthio) pyrimidine, palladium acetate, triphenylphosphine, cesium carbonate, DMF, 80°C, 16h; iii) Oxone, Methanol, H₂O; iv) N-(2-Aminoethyl)benzenesulfonamide or N-Aminoethyl cylic sulfonamide, DIPEA, DMSO, 80°C; v) BBr₃, Dichloromethane; vi) Pd/C, H₂, methanol.

The compound of each reaction scheme is shown in the following Example.

### Example 1. 6-Phenylimidazo[2,1-b]oxazole (3a in Reaction Scheme 2)

To acetonitrile (120 mL), 2-aminooxazole (2 in Reaction Scheme 2, 4.7 g, 47.4 mmol) and α-bromo-4-acetophenone (10.8 g, 49.9 mmol) were added, and stirred at room temperature for 48 hours. Completion of the reaction was confirmed by TLC, followed by filtration. A produced solid was added to toluene (100 mL). 20 mL of titanium chloride (1 M dichloromethane solution) was slowly added to the suspension at 0 °C. This reaction solution was refluxed and stirred for 20 hours, and concentrated to one half of its initial volume. A saturated sodium carbonate solution was added thereto, and extracted with ethyl acetate. An organic layer was dried over anhydrous Na₂SO₄, followed by filtration. The resulting product was distilled under reduced pressure and concentrated to obtain a target compound of 6-phenylimidazo[2,1-b]oxazole (3a) in a crystal state.

White solid (5.1 g, 56 %). mp 143-144°C; IR (KBr) [cm⁻¹]: 3139, 3120, 1955, 1884, 1602. ¹H-NMR (DMSO-d₆, 300 MHz) δ 8.63 (d, 1H, J = 7.17 Hz), 8.29 (t, 1H, J = 5.71 Hz), 7.77 (t, 3H, J = 6.7 Hz), 7.49-7.36 (m, 3H). ¹³CNMR (DMSO-d₆, 75 MHz) δ 158.1, 154.4, 134.9, 132.6, 127.7, 125.7, 121.9, 119.6, 111.5, 104.2.

### Example 2. 5-(2-(Methylthio)pyrimidin-4-yl)-6-phenylimidazo[2,1-b]oxazole (4a in Reaction Scheme 2)

6-Phenylimidazo[2,1-b]oxazole (compound 3a, 8.26 g, 37.8 mmol), 4-chloro-2-(methylthio)pyrimidine (9.09 g, 56.6 mmol), cesium carbonate (18.44 g, 56.6 mmol), palladium acetate (1.71 g, 7.6 mmol), and triphenylphosphine (3.96 g, 15.1 mmol) were added to DMF (80 mL) and stirred at 80 °C for 36 hours. Completion of the reaction was confirmed by TLC, and the reaction solution was cooled to room temperature. EtOAc (200 mL) and distilled water (250 mL) were added to separate layers. An aqueous layer was extracted with EtOAc (100 mL) three times, and discarded. An organic layer was washed with saline (100 mL), and dried over anhydrous Na₂SO₄, followed by filtration. The resulting product was distilled under reduced pressure and concentrated, and then purified by flash column chromatography to obtain 4.37 g of a target compound of 6-phenyl-5-(2-(methylthio)pyrimidin-4-yl)imidazo[2,1-b]oxazole (4a) in a crystal state.

White solid(19%), mp. 143-144 °C; IR (KBr) [cm⁻¹]: 3054, 6103, 1538, 1685; ¹H-NMR (CDCl₃, 300 MHz) δ 8.62 (dd, 1H, J = 4.53, J = 0.7), 8.23 (dd, 1H, J = 5.4, J = 0.7), 7.65-7.62 (m, 2H), 7.47-7.45 (m, 3H), 6.97 (dd, 1H, J = 4.53, J = 0.66), 6.89 (dd, 1H, J = 4.77, J = 0.66), 2.64 (s, 3H); ¹³C NMR (CDCl₃,75 MHz) δ 172.4, 156.2, 156.2, 152.7, 151.1, 134.6, 129.2, 128.9, 128.8, 122.2, 120.1, 112.7, 112.0, 14.2.

### Example 3. 5-(2-(Methylsulfonyl)pyrimidin-4-yl)-6-phenylimidazo[2,1-b]oxazole (5a in Reaction Scheme 2)

6-phenyl-5-(2-(methylthio)pyrimidin-4-yl)imidazo[2,1-b]oxazole (Compound 4a, 3.0 g, 8.8 mmol) was added to MeOH (300 mL). While stirring at room temperature, oxone (14.76 g, 97.0 mmol) in distilled water (60 mL) was added thereto, followed by stirring for 16 hours. Completion of the reaction was confirmed by TLC, and the organic solvent was removed by distillation under reduced pressure. DCM (60 mL) was added to an aqueous layer to separate layers. An aqueous layer was extracted with DCM (30 mL) three times, and the aqueous layer was discarded. An organic layer was washed with saline (50 mL) and distilled water (50 mL), and dried over anhydrous Na₂SO₄, followed by filtration. The resulting product was distilled under reduced pressure and concentrated, and then purified by flash column chromatography to obtain 2.69 g of a target compound of 6-phenyl-5-(2-(methylsulfonyl)pyrimidin-4-yl)imidazo[2,1-b]oxazole (5a) in a crystal state.

White solid(85%); ¹H-NMR (CDCl₃, 300 MHz) δ 8.89 (d, 1H, J = 4.30), 8.56 (d, 1H, J = 5.40), 7.71 (d, 2H, J = 2.50), 7.64-7.45 (m, 3H), 7.42 (d, 1H, J = 5.20), 7.17 (d, 1H, J = 4.40), 3.45 (s, 3H); ¹³C NMR (CDCl₃,75 MHz) δ 165.7, 157.5, 156.8, 153.3, 134.1, 133.3, 130.0, 129.5, 129.1, 129.0, 128.3, 119.5, 117.5, 113.8, 39.3.

### Example 4. 6-(4-Fluorophenyl)-5-(2-(methylsulfonyl)pyrimidin-4-yl)imidazo[2,1-b]oxazole (5c in Reaction Scheme 2)

This compound was synthesized in the same manner as in the synthesis of compound 5a.

Yellow solid (85 %); mp. 115-116 °C. ¹H-NMR (CDCl₃,300MHz) δ 8.90 (d, 1H, J = 4.5 Hz), 8.53 (d, 1H, J = 5.6 Hz) 7.63 (q, 2H, J = 4.7 Hz), 7.33 (d, 1H, J = 5.6 Hz), 7.22 (t, 2H, J = 8.6 Hz), 7.10 (d, 1H, J = 4.5 Hz), 3.37 (s, 3H); ¹³CNMR (CDCl₃,75 MHz) δ 171.1, 165.7, 157.3, 157.0, 154.2, 151.7, 131.0, 130.9, 129.9, 123.1, 119.4, 117.4, 116.4, 116.1, 114.2, 39.2.

### Example 5. 5-(2-(Methylsulfonyl)pyrimidin-4-yl)-6-phenylimidazo[2,1-b]thiazole (5d in Reaction Scheme 3)

This compound was synthesized in the same manner as in the synthesis of compound 5a.

White solid (95%); mp. 176-177 °C. IR (KBr) [cm⁻¹]: 3137, 3110, 2931, 1979, 1902, 1797, 1444, 1373; ¹H-NMR (CDCl₃, 300 MHz) δ 8.99 (d, 1H, J = 4.41), 8.58 (d, 1H, J = 5.40), 7.73 (d, 2H, J = 2.46), 7.66-7.47 (m, 3H), 7.45 (d, 1H, J = 5.22), 7.17 (d, 1H, J = 4.41), 3.47 (s, 3H); ¹³CNMR (CDCl₃, 75 MHz) δ 165.7, 157.5, 156.8, 153.3, 134.1, 133.3, 130.0, 129.5, 129.1, 129.0, 128.3, 119.5, 117.5, 113.8, 39.3; LC-MS: Predicted value calculated with respect to C₁₆H₁₂N₄O₂S₂ m/z: 356. Measured value: 357(M+1)⁺.

### Example 6. 2-Methyl-1,2,5-thiadiazolidine 1,1-dioxide (6a)

N-methylethylendiamine (1.5 g, 20.8 mmol) and sulfuric diimide (2.0 g, 20.8 mmol) were added to a pyridine solvent (20 mL), and refluxed, stirred, and heated for 3 hours. After the reaction was completed, toluene (5 mL) was added to the reactant, followed by concentration and extraction with ethyl acetate. An organic solvent layer was dried over MgSO₄ and filtered to remove the solvent. A residue was purified by column chromatography (EtOAc) to obtain a compound 6a.

White solid (52.3 %). Mp 80 to 82 °C; ¹H-NMR (CDCl₃, 300 MHz) δ7.27 (s, 1H), 3.53-3.19 (m, 2H), 3.42-3.37 (m, 2H), 2.75 (s, 1H); ¹³C NMR (CDCl₃, 75 MHz) δ 32.67, 39.64, 46.94; LC-MS: Predicted value calculated with respect to C₃H₈N₂O₂S m/z: 136. Measured value: 137(M+1)⁺.

### Example 7. Benzyl(2-(5-methyl-1,1-dioxido-1,2,5-thiadiazolidin-2-yl)ethyl) carbamate (7a)

Under nitrogen atmosphere, the compound 6a (0.856 g, 6.26 mmol) was dissolved in 5 mL of anhydrous DMSO, and 60% NaH (0.282 g, 11.8 mmol) was slowly added dropwise at 0°C. The temperature was raised to room temperature, and then the solution was stirred for 1 hour. 2-(((benzyloxy)carbonyl)amino)ethyl methane sulfonate (1.5 g, 5.83 mmol) was slowly added dropwise at 0 °C, and then stirred at room temperature for 5 hours. After the reaction was completed, extraction was performed with ethyl acetate, and an organic solvent layer was dried over MgSO₄. Filtration was performed to remove the solvent, and a residue was purified by column chromatography (EtOAc) to obtain a compound 7a (0.82 g).

Sticky oil (42.9%). ¹H-NMR (CDCl₃, 300MHz) δ7.36-7.30 (m, 5H), 5.19 (bs, 1H), 5.10 (s, 2H), 3.46 (q, 2H, J = 5.9 Hz), 3.36-3.26 (m, 4H), 3.20 (t, 2H, J = 6.0 Hz). LC-MS: Predicted value calculated with respect to C₁₃H₁₉N₃O₄S m/z: 313. Measured value: 314(M+1)⁺.

### Example 8. Synthesis of 2-(2-aminoethyl)-5-methyl-1,2,5-thiadiazolidine 1,1-dioxide (8a)

The compound 7a (0.82 g, 2.5 mmol) was dissolved in 10 mL of methanol, and then 10% Pd/C (0.40 g) was added thereto. Hydrogenation was performed for 2 hours, filtration was performed, and the solvent was removed by distillation under reduced pressure. A product was used in the next reaction without purification (yield: 91%).

### Example 9. N-(2-aminoethyl)-4-fluorobenzenesulfonamide (11b)

White solid(72%); mp. 108 to 109; IR (KBr) cm⁻¹: 3582, 3350, 3299, 3107, 3068, 1903, 1317, 838; ¹H-NMR (CDCl₃, 300 MHz) δ7.84-7.79 (m, 2H), 7.11 (t, 2H, J = 6 Hz), 2.89 (t, 2H, J = 6 Hz), 2.71(t, 2H, J = 6 Hz). ¹³C NMR (CDCl₃, 75 MHz) δ166.7, 136.0, 129.7(J=33), 116.3(J=90), 45.2, 40.9. LC-MS: Predicted value calculated with respect to C₈H₁₁FN₂O₂S m/z: 218 Measured value: 219(M+1)⁺.

### Example 10. 2-(2-((4-(6-(4-Fluorophenyl)imidazo[2,1-b]oxazol-5-yl)pyrimidin-2-yl)amino)ethyl )-5-methyl-1,2,5-thiadiazolidine 1,1-dioxide (11)

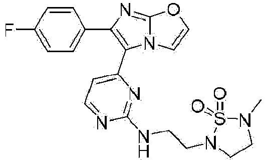

Light yellow solid; to DMSO (5 mL), 6-(4-fluorophenyl)-5-(2-(methylsulfonyl)pyrimidin-4-yl)imidazo[2,1-b]thiazole (compound 5c, 0.19 g, 0.50 mmol), 2-(2-aminoethyl)-5-methyl-1,2,5-thiadiazolidine 1,1-dioxide (compound 8a, 0.27 g, 1.24 mmol), and DIPEA (0.29 mL, 1.70 mmol) were added and stirred at 80 °C for 8 hours. This mixture was cooled to room temperature, and distilled water (10 mL) and EtOAc (10 mL) were added to separate layers. An aqueous layer was extracted with EtOAc (10 mL) twice and discarded. An organic layer was washed with saline (10 mL), and dried over anhydrous Na₂SO₄, followed by filtration. A product was concentrated under reduced pressure, and purified by recrystallization using DCM (1 mL) and hexane (3 mL) to obtain 0.18 g of a target compound 1I in a crystal form.

(72%); ¹H-NMR (CDCl₃, 300MHz) δ 8.52(d, 1H, J = 4.5 Hz), 8.08 (s, 1H), 7.61 (q, 2H, J = 4.6 Hz), 7.13 (t, 2H, J = 8.5 Hz), 6.91 (d, 1H, J = 4.5 Hz), 6.55 (d, 1H, J = 5.5 Hz), 5.81 (bs, 1H), 3.78 (q, 2H, J = 6.1 Hz), 3.43-3.30 (m, 6H), 2.79 (s, 3H);

### Example 11. 2-(2-((4-(6-(3-Methoxyphenyl)imidazo[2,1-b]oxazol-5-yl)pyrimidin-2-yl)amino)eth yl)-5-methyl-1,2,5-thiadiazolidine 1,1-dioxide (41)

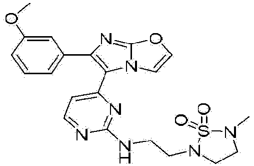

This compound was synthesized in the same manner as in the synthesis of compound 1I.

White solid (50.5%); mp 144-145 °C; ¹HNMR (CDCl₃, 300 MHz) 8.58 (d, 1H, J = 4.5 Hz), 8.06 (d, 1H, J = 5.3 Hz), 7.33 (t, 1H, J = 7.8 Hz), 7.23-7.19 (m, 2H), 6.98-6.90 (m, 2H), 6.58 (d, 1H, J = 5.4 Hz), 3.83 (s, 3H), 3.78 (q, 2H, J = 6.1 Hz), 3.43-3.30 (m, 6H), 2.78 (s, 3H).

### Example 12. 2-(2-((4-(6-(3-Hydroxylphenyl)imidazo[2,1-b]oxazol-5-yl)pyrimidin-2-yl)amino)et hyl)-5-methyl-1,2,5-thiadiazolidine 1,1-dioxide (111)

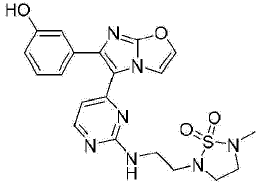

Under nitrogen atmosphere, the compound 4I (17.5 mg, 0.1 mmol) was dissolved in 3 mL of MC, and then BBr₃ (0.02 mL of 1 M solution in MC, 0.3 mmol) was added dropwise at -78 °C, and stirred at the same temperature for 30 minutes, and further stirred at room temperature for 1 hour. After the reaction was completed, a product was dissolved with ethyl acetate, and washed with NaHCO₃ and H₂O. The organic solvent was dried over MgSO₄, followed by filtration. A product was purified by column chromatography (EtOAc:hexane = 1:2) to obtain a compound 1II.

White solid (78.1 %); mp 151-152 °C; ¹HNMR (DMSO-d₆, 300 MHz) δ 9.56 (s, 1H), 8.12 (d, 1H, J = 5.3 Hz), 7.56-7.45 (m, 2H), 7.25 (t, 1H, J = 7.9 Hz), 7.00 (s, 1H), 6.97 (d, 2H, J = 1.5 Hz), 6.84-6.80 (m, 1H), 6.41 (d, 1H, J = 5.3 Hz), 3.71-3.62 (m, 2H), 3.48-3.23 (m, 4H), 3.16 (t, 2H, J = 6.0 Hz), 2.60 (s, 3H).

### Example 13. 2-Ethyl-5-(2-((4-(6-(3-hydroxylphenyl)imidazo[2,1-b]oxazol-5-yl)pyrimidin-2-yl)a mino)ethyl)-1,2,5-thiadiazolidine 1,1-dioxide (2II)

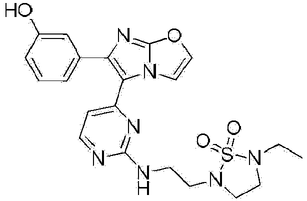

This compound was synthesized in the same manner as in the synthesis of compound 1II.

White solid (66.5 %); mp 149-150 °C; ¹HNMR (DMSO-d₆, 300 MHz) δ 9.58 (s, 1H), 8.11 (d, 1H, J = 5.4 Hz), 7.56-7.45 (m, 2H), 7.25 (t, 1H, J = 8.0 Hz), 6.99-6.80 (m, 3H), 6.41 (d, 1H, J = 5.3 Hz), 3.55-3.49 (m, 2H), 3.44-3.23 (m, 4H), 3.19-3.15 (m, 2H), 2.95 (q, 2H, J = 7.2 Hz).

### Example 14. 2-(2-((4-(6-(3-Hydroxylphenyl)imidazo[2,1-b]oxazol-5-yl)pyrimidin-2-yl)amino)et hyl)-4,4-dimethyl-1,2,5-thiadiazolidine 1,1-dioxide (5II)

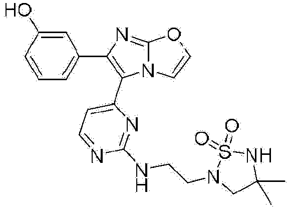

This compound was synthesized in the same manner as in the synthesis of compound 1II.

White solid (25.0 %); mp 196-197 °C; ¹HNMR (CDCl₃, 400 MHz) δ 8.55 (d, 1H, J = 5.4 Hz), 8.04 (d, 1H, J = 5.4 Hz), 7.30-7.28 (m, 1H), 7.15-7.13 (m, 2H), 6.95 (d, H, J = 8.1 Hz), 3.76 (t, 2H, J = 6.1 Hz), 3.33 (t, 2H, J = 5.92 Hz), 3.25 (s, 2H), 1.45 (s, 6H).

### Example 15. 2-(2-((4-(6-(3-Hydroxylphenyl)imidazo[2,1-b]oxazol-5-yl)pyrimidin-2-yl)amino)et hyl)-6-methyl-1,2,6-thiadiazinane 1,1-dioxide (6II)

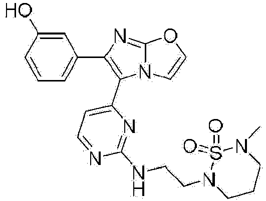

This compound was synthesized in the same manner as in the synthesis of compound 1II.

White solid (71.7 %); mp 184-185 °C; ¹HNMR (CDCl₃, 400 MHz) δ 8.57 (d, 1H, J = 4.5 Hz), 8.01 (d, 1H, J = 5.4 Hz), 7.28-7.26 (m, 1H), 7.15-7.11 (m, 2H), 6.88 (d, H, J = 7.7 Hz), 6.59 (d, 1H, J = 5.3 Hz), 3.67 (t, 2H, J = 5.4 Hz), 3.48 (t, 2H, J = 5.2Hz), 3.37 (t, 2H, J = 6.0 Hz), 2.75 (s, 3H), 1.78 (m, 2H).

### Example 16. 2-(2-((4-(6-(3-Hydroxylphenyl)imidazo[2,1-b]oxazol-5-yl)pyrimidin-2-yl) amino) ethyl)-1,2,6-thiadiazinane 1,1-dioxide (8II)

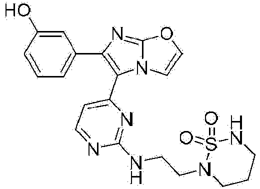

This compound was synthesized in the same manner as in the synthesis of compound 1II.

White solid (85.3 %); mp 183-184 °C; ¹H-NMR(CDCl₃, 400 MHz) δ 8.85 (s, 1H), 8.14 (d, 1H, J = 5.9 Hz), 7.78 (d, 1H, J = 4.1 Hz), 7.47 (t, 1H, J = 7.7 Hz), 7.17-7.09 (m, 3H), 6.74 (d, H, J = 6.7 Hz), 3.88 (m, 2H), 3.60 (t, 2H, J = 7.7 Hz), 3.44-3.41 (m, 4H), 1.78 (m, 2H).

### Example 17. 4-Fluoro-N-(2-((4-(6-phenylimidazo[2,1-b]thiazol-5-yl)pyrimidin-2-yl)amino)ethyl) benzenesulfonamide (1111)

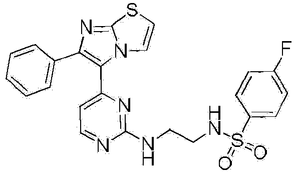

Under nitrogen atmosphere, the compound 5d (327 mg, 0.92 mmol), N-(3-aminoethyl)-4-fluorobenzenesulfonamide (11b, 540 mg, 2.48 mmol), and diisopropylethylamine (0.57 mL 3.3 mmol) were added to a DMSO solvent (10 mL), and stirred at 80°C for 8 hours. After the reaction was completed, a product was extracted with ethyl acetate. An organic solvent layer was dried over MgSO₄, and filtered to remove the solvent. A residue was purified by column chromatography (EtOAc) to obtain a compound 1III.

Orange color solid (52.1 %); mp. 121-122 °C; ¹HNMR (DMSO-d₆, 300 MHz) δ 8.83 (bs, NH), 8.05 (d, 1H, J = 6 Hz), 7.80 (dd, 2H, J = 6, J = 3 Hz), 7.58 (dd, 4H, J = 6, J = 3 Hz), 7.46 (d, 5H, J = 6 Hz), 6.31 (d, 1H, J = 6 Hz), 2.96 (t, 2H, J = 6 Hz), 2.50 (s, 2H); 13C-NMR(DMSO-d6, 75 MHz) δ 164.2, 162.5, 160.9, 158.2, 156.2, 151.6, 148.0, 140.9, 132.7, 131.7, 129.6, 126.8, 121.0, 115.9, 114.5, 105.7, 38.7, 29.5; LC-MS: Predicted value calculated with respect to C₂₃H₁₉FN₆O₂S₂ m/z: 494 Measured value: 495 (M+1)⁺.

### Example 18. 4-Methoxy-N-(2-((4-(6-phenylimidazo[2,1-b]thiazol-5-yl)pyrimidin-2-yl)amino)eth yl)benzenesulfonamide (2III)

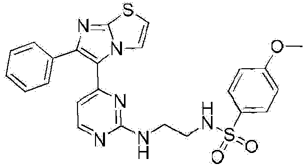

This compound was synthesized in the same manner as in the synthesis of compound 1III.

White solid (65%); mp. 199-200 °C; ¹H-NMR (MeOD, 400 MHz) δ 9-10 (bs, 2NH protons), 8.87(d, 1H, J = 4.8 Hz), 8.50 (d, 2H, J = 7.5 Hz), 8.40 (s, 2H), 8.28 (s, 5H), 8.16 (d, 2H, J = 7.2 Hz), 7.12 (s, 1H), 3.76 (s, 3H), 3.33 (s, 2H), 3.15 (s, 1H); ¹³C NMR (DMSO-d₆, 100 MHz) δ 163.8, 162.5, 161.4, 158.1, 156.2, 151.6, 148.1, 142.9, 138.0, 131.7, 130.1, 127.0, 121.0, 115.9, 114.4, 105.7, 55.3, 41.7, 29.5. LC-MS: Predicted value calculated with respect to C₂₄H₂₂N₆O₂S₂ m/z: 506 Measured value: 507 (M+1)⁺.

### Example 19. N-(2-((4-(6-(3-methoxyphenyl)imidazo[2,1-b]thiazol-5-yl)pyrimidin-2-yl)amino)et hyl)-4-(trifluoromethyl)benzenesulfonamide (8III)

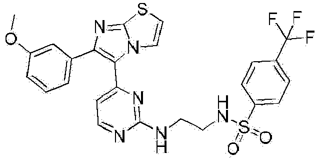

This compound was synthesized in the same manner as in the synthesis of compound 1III.

Colorless sticky oil (25%); ¹HNMR (DMSO-d₆, 400 MHz) δ 8.05 (d, 1H, J = 5.2 Hz), 7.73 (t, 2H, J = 7.2 Hz), 7.59 (dd, 2H, J = 2, J = 8 Hz), 7.47 (d, 4H, J = 7.2 Hz), 7.06 (d, 2H, J = 8.8 Hz), 6.31 (d, 1H, J = 5.2 Hz), 3.79 (s, 3H), 3.39 (bs, 2H), 2.94 (brs, 2H). LC-MS: Predicted value calculated with respect to C₂₅H₂₁F₃N₆O₃S₂ m/z: 574. Measured value: 575(M+1)⁺.

### Example 20. 4-Fluoro-N-(2-((4-(6-(3-hydroxylphenyl)imidazo[2,1-b]thiazol-5-yl)pyrimidin-2-yl) amino)ethyl)benzenesulfonamide (25III)

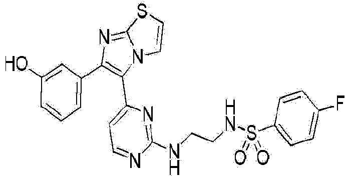

This compound was synthesized in the same manner as in the synthesis of compound 1III.

Dark orange color solid (44%); mp 70-71 °C; IR (KBr) [cm⁻¹]: 3115, 2854, 1639, 1574, 1445, 1328; ¹H-NMR (MeOD, 300) δ 8.68 (s, 1H), 7.87 (t, 3H, J = 9 Hz), 7.58-7.45 (m, 6 H), 6.97 (d, 3H, J = 6 Hz), 6.65 (d, 1H, J = 9 Hz), 3.33 (brs, 2H), 3.22 (t, 2H, J=6). LC-MS: Predicted value calculated with respect to C₂₃H₂₀N₆O₃S₂ m/z: 492. Measured value: 493(M+1)⁺.

### Example 21. 4-Fluoro-N-(3-((4-(6-(3-hydroxylphenyl)imidazo[2,1-b]thiazol-5-yl)pyrimidin-2-yl) amino)propyl)benzenesulfonamide (31III)

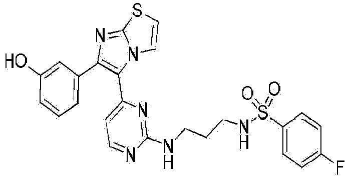

This compound was synthesized in the same manner as in the synthesis of compound 1II.

Lemon yellow color solid (20%); mp 92-93 °C; IR (KBr) [cm⁻¹]: 3382, 3258, 3118, 2933, 1731, 1574, 1447, 1331; 1H-NMR (DMSO-d6, 400) δ 9.59 (s, 1H), 8.08 (d, 1H, J = 4 Hz), 7.85 (dd, 2H, J = 4, J = 8 Hz), 7.70 (t, 1H, J = 4 Hz), 7.45 (d, 1H, J = 4 Hz), 7.40 (t, 3H, J = 8 Hz), 7.27 (t, 1H, J = 8 Hz), 6.98 (d, 1H, J = 4 Hz), 6.84 (d, 1H, J = 8 Hz), 6.38 (d, 1H, J = 4 Hz), 3.31 (d, 2H, J = 4 Hz), 2.85 (q, 2H, J = 4 Hz), 1.70 (t, 2H, J = 4 Hz). LC-MS: Predicted value calculated with respect to C₂₄H₂₁FN₆O₃S₂ m/z: 524. Measured value: 525(M+1)⁺.

### Example 22. 6-(4-fluoro-3-methoxyphenyl)imidazo[2,1-b]thiazole(3j in Reaction Scheme 5)

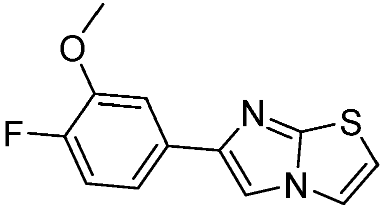

A mixture of 2-bromo-1-(4-fluoro3-methoxyphenyl)ethan-1-one (44.6 mmol) and 2-aminothiazole (5, 4.46g, 44.6 mmol, 1 Eq) in absolute ethanol (60 ml) was dissolved, stirred and refluxed for 18h. The reaction mixture was concentrated under reduced pressure. Ice-cold water (50 ml) was added followed by ammonia solution (30%, 100 ml). The reaction mixture was stirred at rt for 2 h. The formed precipitate was filtered off, washed with water (2 × 20 ml) and dried to give the crude solid product. The crude product was purified by column chromatography.

### Example 23. 6-(4-fluoro-3-methoxyphenyl)-5-(2-(methylthio)pyrimidin-4-yl)imidazo[2,1-b]thia zole(4j in Reaction Scheme 5)

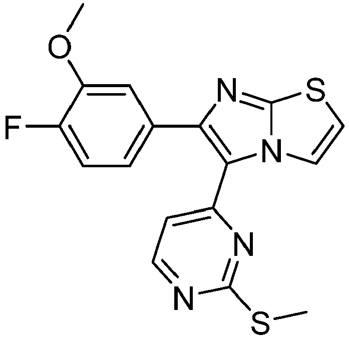

In a three neck flask, 4-chloro-2-(methylthio)pyrimidine (7, 70.7 mg, 0.44 mmol, 1 Eq), potassium carbonate (60.8 mg, 0.44 mmol, 1 Eq), palladium acetate (19.8 mg, 0.09 mmol, 0.2 Eq) and triphenylphosphine (34.6 mg, 0.13 mmol, 0.3 Eq) were mixed with compound 6-(4-fluoro-3-methoxyphenyl)imidazo[2,1-b]thiazole (0.44 mmol, 1 Eq). Air was replaced by nitrogen. Anhydrous DMF (10 ml) was added and the mixture was purged with nitrogen several times. The reaction mixture was stirred at 80 °C for 18 h. The reaction mixture was cooled and extracted between EA (20 ml) and water (10 ml). The organic layer was separated, dried over anhydrous sodium sulfate and evaporated. The crude residue was used in the next step without further purification.

### Example 24. 6-(4-fluoro-3-methoxyphenyl)-5-(2-(methylsulfonyl)pyrimidin-4-yl)imidazo[2,1-b] thiazole(5j in Reaction Scheme 5)

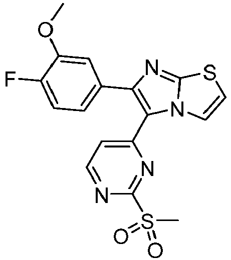

To a solution of 6-(4-fluoro-3-methoxyphenyl)-5-(2-(methylsulfonyl)pyrimidin-4-yl)imidazo[2,1-b]thiazo le (0.44 mmol) in methanol (10 ml), a solution of oxone (0.9 g, 1.32 mmol) in water (10 ml) was added dropwise at rt. The mixture was stirred at rt for 48 h. The reaction mixture was concentrated under reduced pressure. The reaction mass was extracted with dichloromethane (20 ml) and the organic layer was separated. The aqueous layer was extracted with dichloromehane (3 × 10 ml). The combined organic layers were dried over anhydrous sodium sulfate and evaporated under reduced pressure. The crude residue was purified by column chromatography.

### Example 25. 4-Fluoro-N-(3-((4-(6-(4-fluoro-3-methoxyphenyl)imidazo[2,1-b]thiazol-5-yl)pyrimi din-2-yl)amino)propyl)benzenesulfonamide (37III)

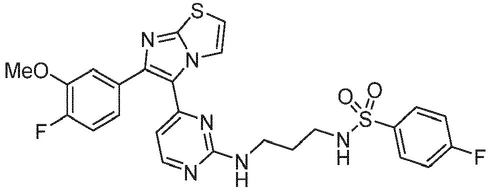

To a solution of 6-(4-fluoro-3-methoxyphenyl)-5-(2-(methylsulfonyl)pyrimidin-4-yl)imidazo[2,1-b]thiazo le (0.26 mmol) in DMSO (3 ml), N-(3-aminopropyl)-4-fluorobenzenesulfonamide (0.39 mmol) and DIPEA (300 mg, 2.34 mmol) were added. The reaction mixture was stirred at 100°C for 18h. The reaction mixture was cooled and extracted between EA (20 ml) and water (10 ml)). The organic layer was dried over anhydrous sodium sulfate and evaporated under reduced pressure. The crude residue was purified by column chromatography.

Yield: 70%. 1H NMR (400 MHz, DMSO-d6) δ 8.08 (d, J = 8.0 Hz, 1H), 7.88 (q, J = 4.0 Hz, 3H), 7.69 (s, 1H), 7.43-7.37 (m, 4H), 7.22 (t, J = 4.0 Hz, 1H), 6.54 (s, 1H), 3.86 (s, 3H), 2.91 (d, J = 8.0 Hz, 2H), 2.52 (s, 2H), 1.81 (d, J = 4.0 Hz, 2H). LC/MS 558 (M+ +1).

### Example 26. 4-Fluoro-N-(3-((4-(6-(4-fluoro-3-hydroxyphenyl)imidazo[2,1-b]thiazol-5-yl)pyrimi din-2-yl)amino)propyl)benzenesulfonamide (47III):

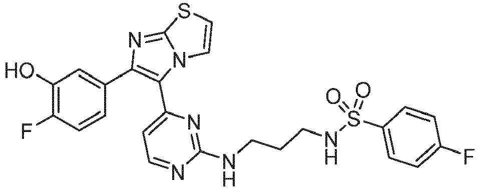

To a mixture of 4-Fluoro-N-(3-((4-(6-(4-fluoro-3-methoxyphenyl)imidazo[2,1-b]thiazol-5-yl)pyrimidin-2 -yl)amino)propyl)benzenesulfonamide (0.1 mmol) in methylene chloride (5mL), BBr₃ (0.13 g, 1.0 mmol) was added dropwise at -78 °C under nitrogen, and the reaction mixture was stirred at 0 °C for 24 h. The mixture was quenched with saturated aqueous NaHCO₃. Ethyl acetate (10 mL) was added and the organic layer was separated. The aqueous layer was extracted with ethyl acetate (3 × 10 mL). The combined organic layer extracts were washed with brine and dried over anhydrous Na₂SO₄. The organic solvent was evaporated under reduced pressure, and the residue was purified by column chromatography.

Yield: 40%. 1H NMR (400 MHz, MeOD) δ 8.78 (s, 1H), 8.03 (d, J = 5.2 Hz, 1H), 8.89 (q, J = 5.2 Hz, 4H), 7.34-7.24 (m, 5H), 7.19-7.15 (m, 2H), 7.05-7.01 (m, 1H ), 6.45 (d, J = 5.6 Hz, 1H), 3.02 (t, J = 6.8 Hz, 2H), 2.89 (t, J = 6.8 Hz, 2H), 1.83 (t, J = 6.8 Hz, 2H. LC/MS 543 (M+ +1).

### Example 27. 6-(3-nitrophenyl)imidazo[2,1-b]thiazole(3I in Reaction Scheme 5):

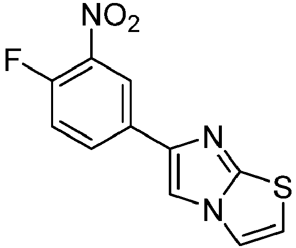

A solution of compound 1i (1 g, 4.1 mmol, 1 Eq) and compound 2 (0.5 g, 4.9 mmol, 1.2 Eq) in MeOH (50 ml) was stirred under reflux for 18 h. The organic solvent was evaporated under vacuum. The crude solid ppt was stirred in NH₄OH solution at rt for 2 h. The crude solid product was filtered and washed with cold water (3 × 100 ml) and dried to give the title compound .

Yield: 90%. m.p.: 167-9 °C. 1H NMR (400 MHz, DMSO-d6) δ 8.63 (t, J = 2.2 Hz, 1H, Ar-H), 8.27-8.24 (m, 1H, Ar-H), 8.09-8.06 (m, 1H, Ar-H), 7.98 (d, J = 4.4 Hz, 1H, Ar-H), 7.67 (t, J = 8.2 Hz, 1H, Ar-H), 7.32 (d, J = 4.4 Hz, 1H, Ar-H). 13C NMR (100 MHz, DMSO- DMSO-d6) δ 150.25 (Ar-C), 148.78 (Ar-C), 144.40 (Ar-C), 136.46 (Ar-C), 131.26 (Ar-C), 130.61 (Ar-C), 121.87 (Ar-C), 120.52 (Ar-C), 119.32 (Ar-C), 114.39 (Ar-C), 111.44 (Ar-C).

### Example 28. 5-(2-(methylthio)pyrimidin-4-yl)-6-(3-nitrophenyl)imidazo[2,1-b]thiazole (4I in Reaction Scheme 5):

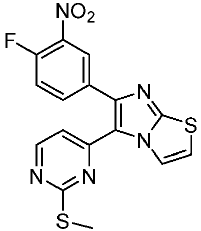

A solution compound 6-(3-nitrophenyl)imidazo[2,1-b]thiazole (0.7 g, 4.1 mmol, 1 Eq) in anhydrous DMF (5 ml) was added dropwise to a mixture of Chloro methylthipyrimidine (1 g, 4.1 mmol, 1 Eq), Ph₃P (0.3 g, 1.3 mmol, 0.3 Eq), K₂CO₃ (0.6 g, 4.1 mmol, 1 Eq) and Pd(OAc)₂ (0.2 g, 0.8 mmol, 0.2 Eq) in anhydrous DMF (10 ml). The reaction mixture was stirred at 80 °C for 8 h. The reaction mixture was cooled and stirred with crushed ice (30 g). The crude ppt was filtered. The crude solid residue was stirred in MeOH (50 ml) at 60 °C for 1 h. The solid product was filtered and washed with MeOH (3 × 20 ml) and dried to give the title compound.

Yield: 30%. m.p.: 187-9 °C. 1H NMR (400 MHz, CDCl3) δ 8.58 (d, J = 4.4 Hz, 2H, Ar-H), 8.34-8.31 (m, 2H, Ar-H), 8.02 (d, J = 8.0 Hz, 1H, Ar-H), 7.66 (t, J = 8.0 Hz, 1H, Ar-H), 7.06 (d, J = 4.4 Hz, 1H, Ar-H), 6.86 (d, J = 5.6 Hz, 1H, Ar-H), 2.66 (s, 3H, SCH₃). 13C NMR (100 MHz, CDCl3) δ 173.15 (Ar-C), 156.83 (Ar-C), 155.66 (Ar-C), 153.01 (Ar-C), 148.57 (Ar-C), 147.65 (Ar-C), 136.32 (Ar-C), 135.04 (Ar-C), 129.83 (Ar-C), 124.14 (Ar-C), 123.53 (Ar-C), 121.96 (Ar-C), 120.82 (Ar-C), 113.65 (Ar-C), 112.00 (Ar-C), 14.23 (SCH₃). LC/MS 371 (M+1)⁺.

### Example 29. 5-(2-(methylthio)pyrimidin-4-yl)-6-(3-nitrophenyl)imidazo[2,1-b]thiazole (5I in Reaction Scheme 5):

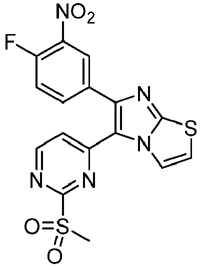

A solution of potassium peroxymonosulfate (5 g, 8.1 mmol, 3 Eq) in water (20 ml) was added dropwise to a solution of 5-(2-(methylthio)pyrimidin-4-yl)-6-(3-nitrophenyl)imidazo[2,1-b]thiazole (1 g, 2.7 mmol, 1 Eq) in MeOH (50 ml). The reaction mixture was stirred at rt for 9 h. The organic solvent was evaporated under vacuum. The crude residue was extracted between DCM (70 ml) and water (30 ml). The organic layer was washed with brine solution (3 × 30 ml). The organic layer was dried over anhydrous Na₂SO₄ and evaporated under reduced pressure. The crude solid residue was purified through column chromatography (hexane, EtOAc; 2, 1) to give the title compound.

Yield: 80%. m.p.: 215-6 °C. 1H NMR (400 MHz, CDCl3) δ 8.91 (d, J = 4.4 Hz, 1H, Ar-H), 8.61 (t, J = 5.6 Hz, 2H, Ar-H), 8.39-8.37 (m, 1H, Ar-H), 8.04 (d, J = 8.0 Hz, 1H, Ar-H), 7.75 (t, J = 7.6 Hz, 1H, Ar-H), 7.35 (d, J = 5.6 Hz, 1H, Ar-H), 7.17 (d, J = 4.8 Hz, 1H, Ar-H), 3.42 (s, 3H, SO₂CH₃). 13C NMR (100 MHz, CDCl₃) δ 166.13 (Ar-C), 157.49 (Ar-C), 154.59 (Ar-C), 149.59 (Ar-C), 148.70 (Ar-C), 135.71 (Ar-C), 130.32 (Ar-C), 124.21 (Ar-C), 123.16 (Ar-C), 120.01 (Ar-C), 117.48 (Ar-C), 114.79 (Ar-C), 39.27 (SO₂CH₃).

### Example 30. N-(3-((4-(6-(3-amino-4-fluorophenyl)imidazo[2,1-b]thiazol-5-yl)pyrimidin-2-yl)am ino)propyl)-4-fluorobenzenesulfonamide (52III):

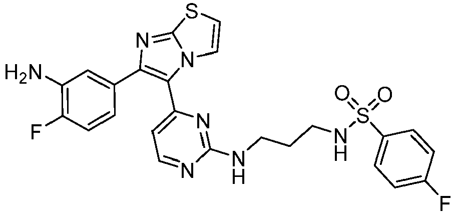

To a solution of 6-(4-fluoro-3-nitro)-5-(2-(methylsulfonyl)pyrimidin-4-yl)imidazo[2,1-b]thiazole (0.26 mmol) in DMSO (3 ml), N-(3-aminopropyl)-4-fluorobenzenesulfonamide (0.39 mmol) and DIPEA (300 mg, 2.34 mmol) were added. The reaction mixture was stirred at 100 °C for 18 h. The reaction mixture was cooled and extracted between EA (20 ml) and water (10 ml). The organic layer was dried over anhydrous sodium sulfate and evaporated under reduced pressure. The crude residue was dissolved in 10 ml methanol and 10% pd/C was added and the mixture was stirred overnight under hydrogen atmosphere. After complete reaction the pd/C was filtered off and the methanol was evaporated. The residue was purified using column chromatography.

1H NMR (400 MHz, MeOH) δ 8.74 (s, 1H), 7.98 (d, J = 4.0 Hz, 2H), 7.91-7.87 (m, 2H), 7.31-7.23 (m, 3H), 7.09-7.03 (m, 2H), 6.83-6.79 (m, 1H), 6.45 (d, J = 4.0 Hz, 1H), 4.47 (s, 2H ), 3.01 (t, J = 4.0 Hz, 2H), 1.81 (t, J = 8.0 Hz, 2H). 13C NMR (100 MHz, MeOH) δ 166.23, 163.01, 162.01, 156.25, 153.24, 151.64, 150.01, 149.11, 136.23, 130.11, 129.78, 120.12, 118.15, 117.12, 115.52114.81, 105.91, 40.35, 38.19, 29.1

The other compounds were synthesized in the same manner as in the synthesis of compound 11, compound 111, or compound III, except that different substituents were used. Examples of the synthesized compounds are as shown in the following Table 1. Only compounds (37III), (38III), (42III), (43III), (46III), (48III), (50III), (52III), (60IV), (64IV), (65IV), (70IV), (72IV) and (73IV) are part of the invention.

**[Table 1]**

| Compound No. | Structure | Compound No. | Structure |
|---|---|---|---|
| 1I | 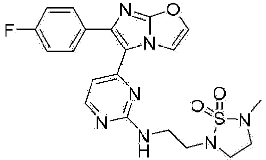 | 2I | 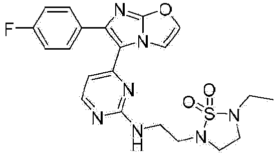 |
| 3I | 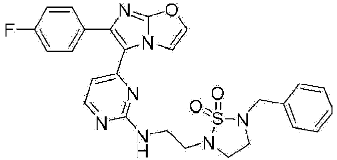 | 4I | 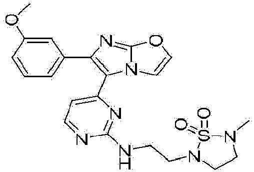 |
| 5I | 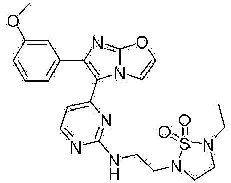 | 6I | 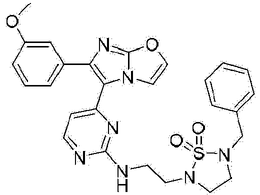 |
| 7I | 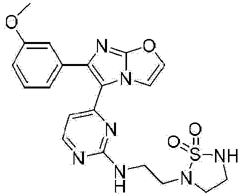 | 8I | 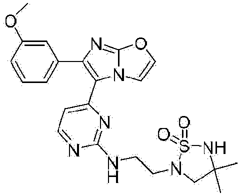 |
| 9I | 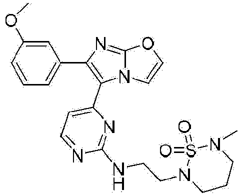 | 10I | 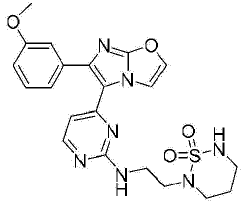 |
| 11I | 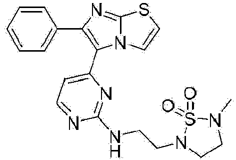 | 12I | 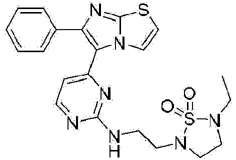 |
| 13I | 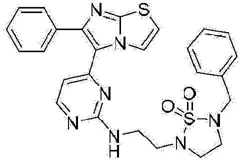 | 14I | 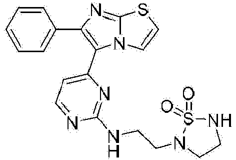 |
| 15I | 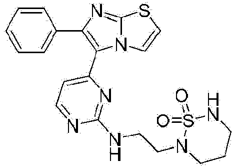 | 16I | 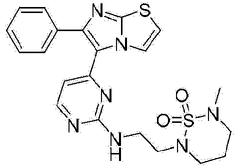 |
| 17I | 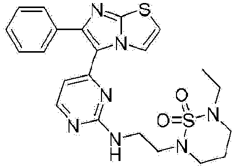 | 18I | 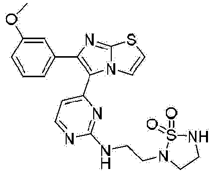 |
| 19I | 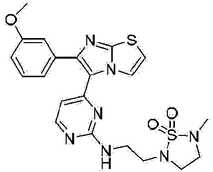 | 20I | 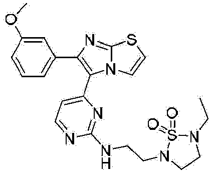 |
| 21I | 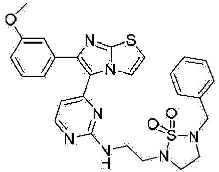 | 22I | 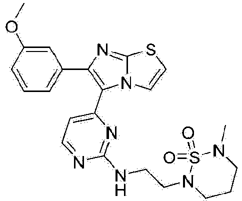 |
| 23I | 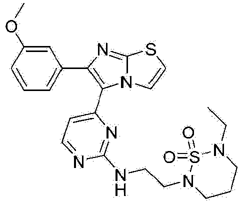 | 24I | 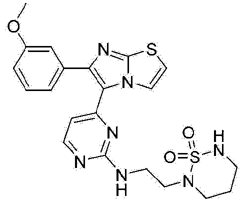 |
| 25I | 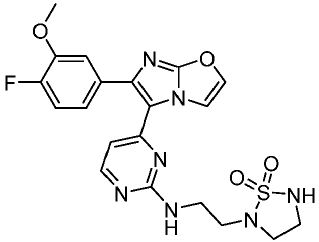 | 26I | 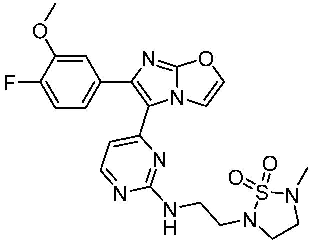 |
| 27I | 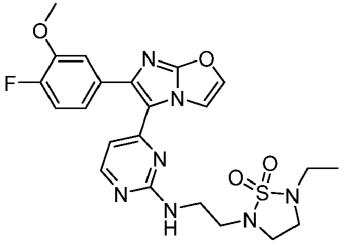 | 28I | 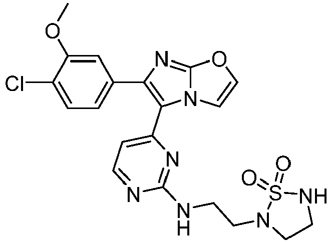 |
| 29I | 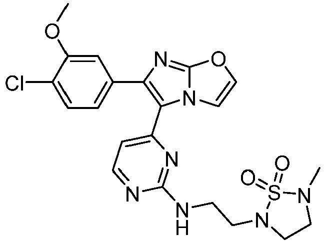 | 30I | 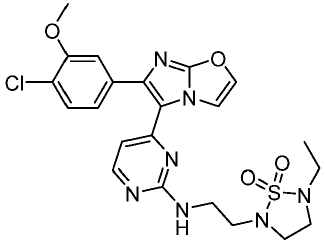 |
| 31I | 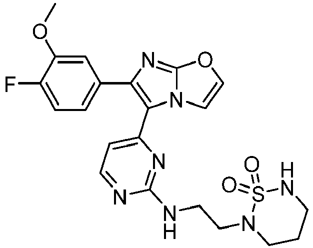 | 32I | 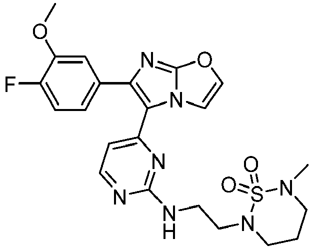 |
| 33I | 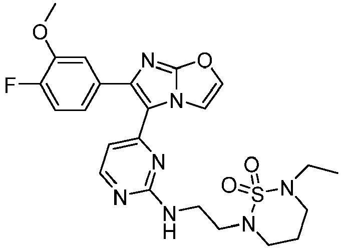 | 34I | 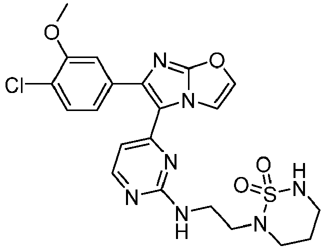 |
| 35I | 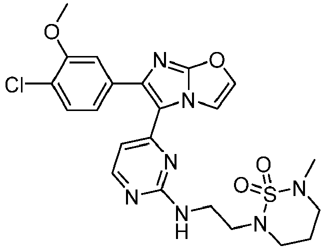 | 36I | 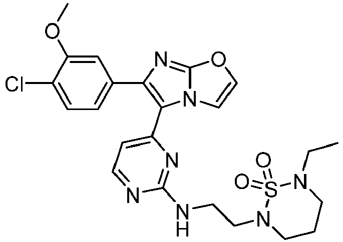 |
| 37I | 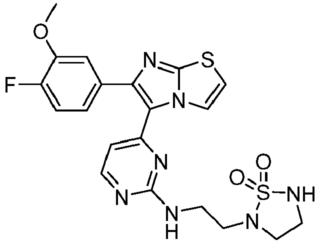 | 38I | 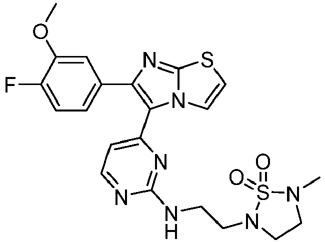 |
| 39I | 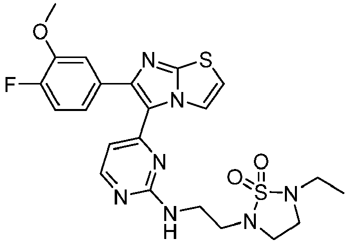 | 40I | 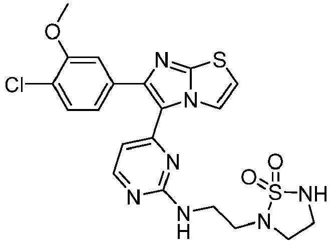 |
| 41I | 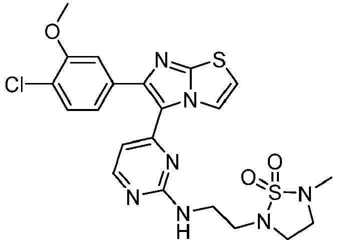 | 42I | 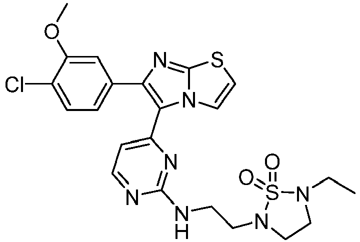 |
| 43I | 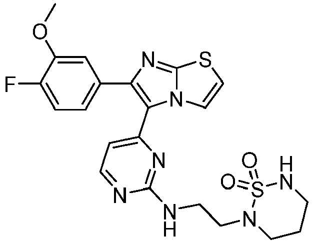 | 44I | 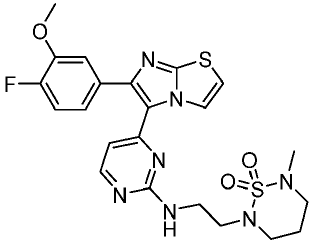 |
| 45I | 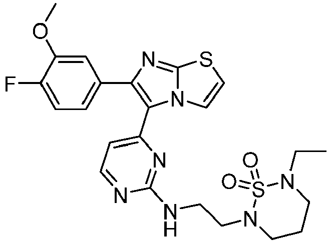 | 46I | 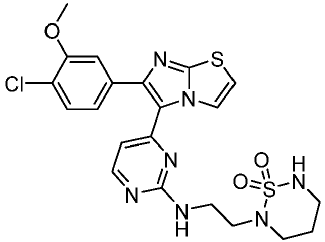 |
| 47I | 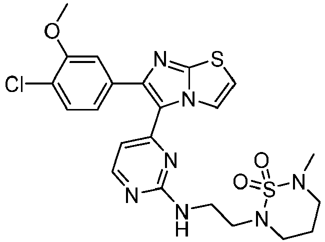 | 48I | 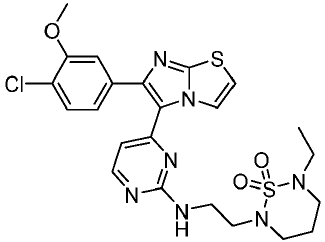 |
| 11I | 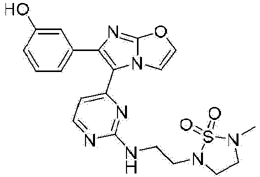 | 2II | 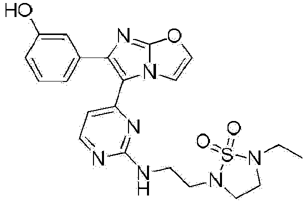 |
| 3II | 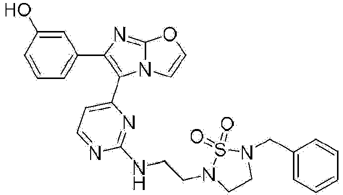 | 4II | 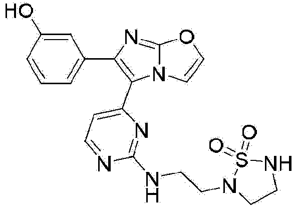 |
| 5II | 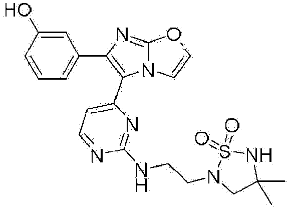 | 6II | 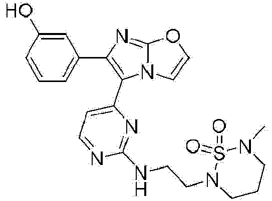 |
| 7II | 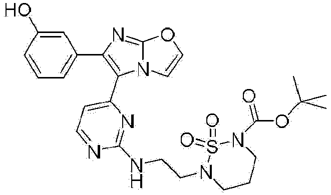 | 8II | 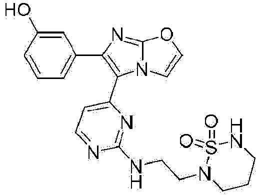 |
| 9II | 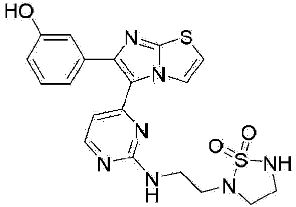 | 10 II | 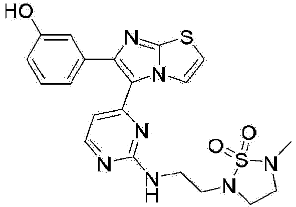 |
| 11II | 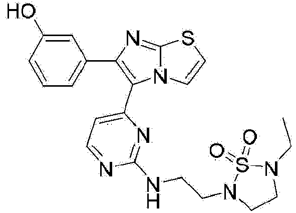 | 12 II | 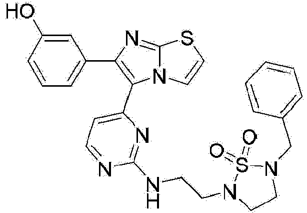 |
| 13 II | 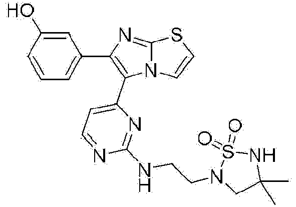 | 14 II | 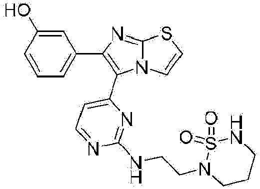 |
| 15 II | 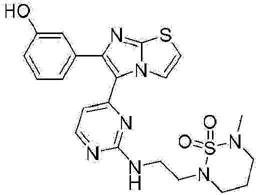 | 16 II | 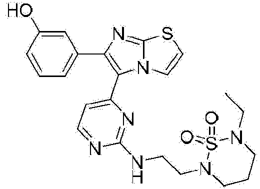 |
| 17 II | 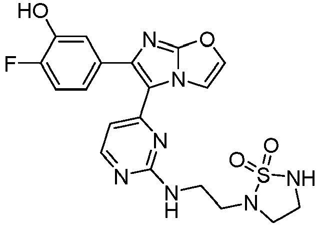 | 18 II | 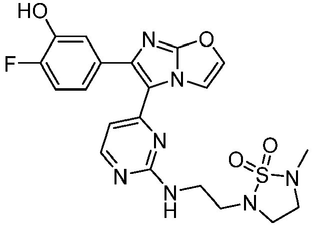 |
| 19 II | 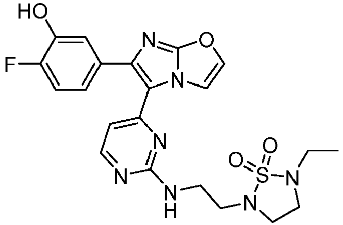 | 20 II | 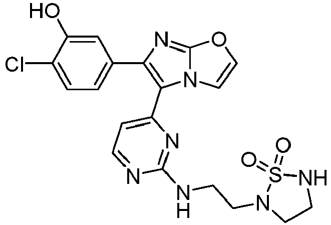 |
| 21 II | 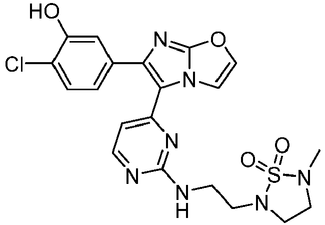 | 22 I | 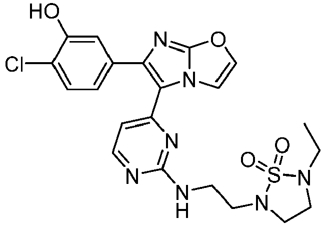 |
| 23II | 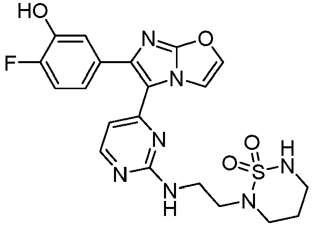 | 24 II | 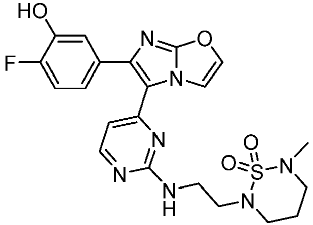 |
| 25II | 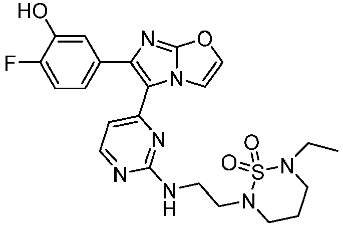 | 26 II | 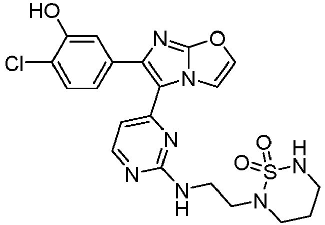 |
| 27II | 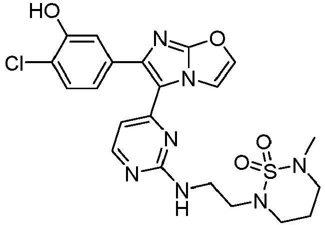 | 28 II | 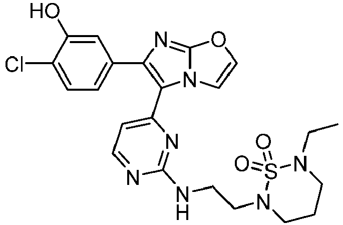 |
| 29II | 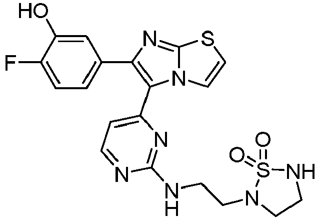 | 30 II | 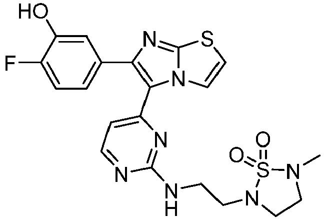 |
| 31 II | 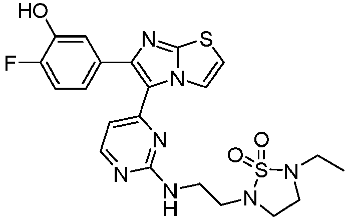 | 32 II | 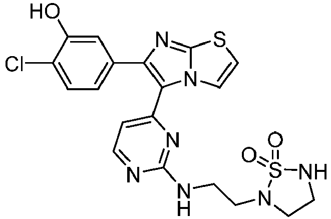 |
| 33II | 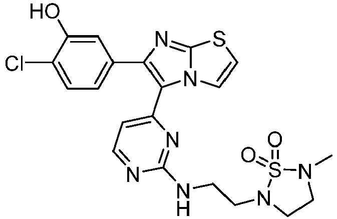 | 34 II | 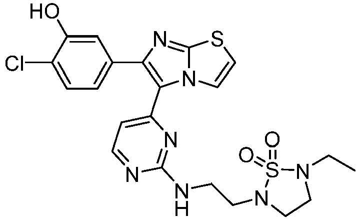 |
| 35II | 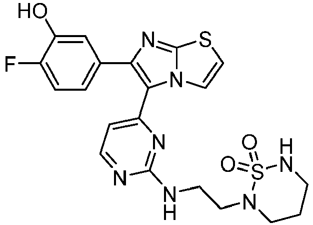 | 36 II | 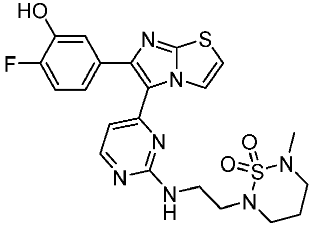 |
| 37II | 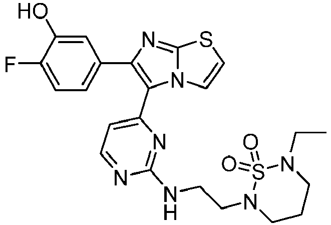 | 38 II | 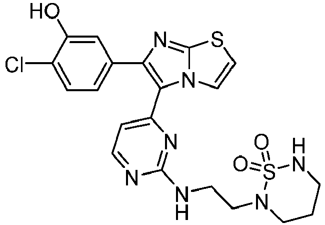 |
| 39II | 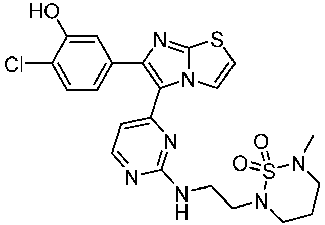 | 40 II | 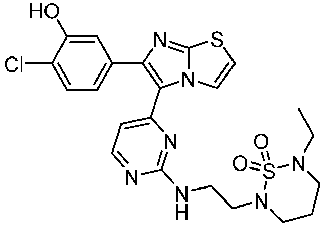 |
| 1III | 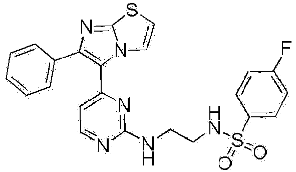 | 2 III | 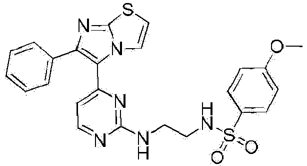 |
| 3III | 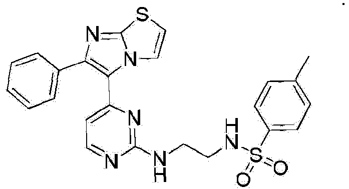 | 4III | 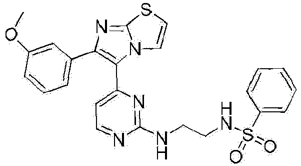 |
| 5III | 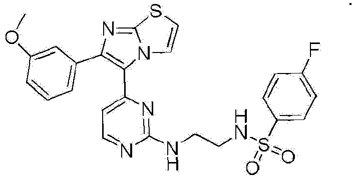 | 6III | 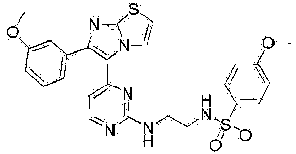 |
| 7III | 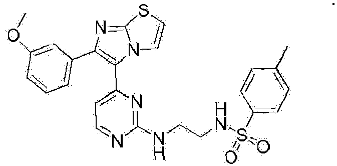 | 8III | 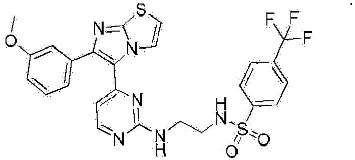 |
| 9III | 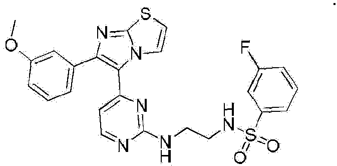 | 10III | 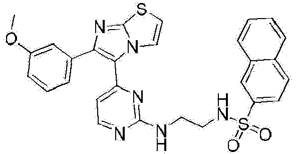 |
| 11III | 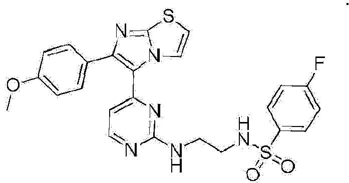 | 12III | 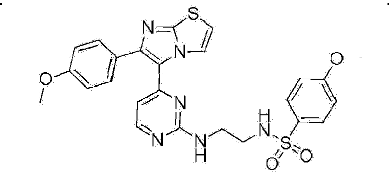 |
| 13III | 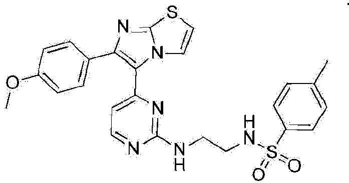 | 14III | 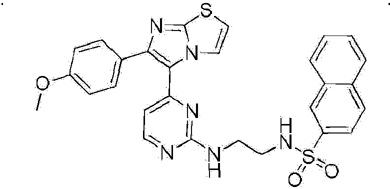 |
| 15III | 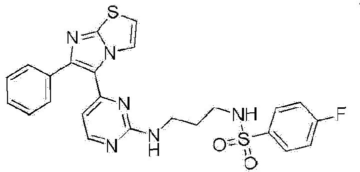 | 16III | 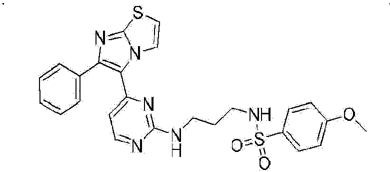 |
| 17III | 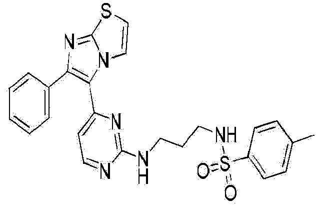 | 18III | 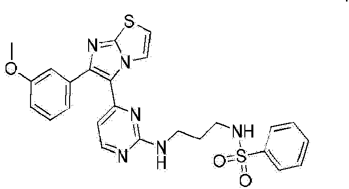 |
| 19III | 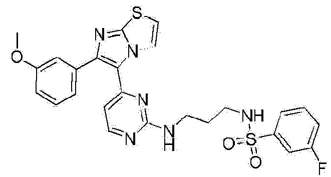 | 20III | 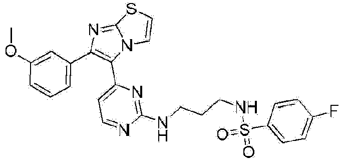 |
| 21III | 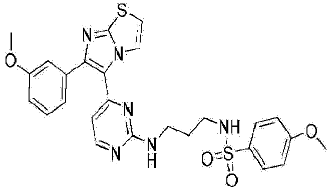 | 22III | 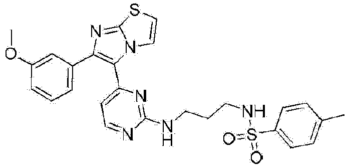 |
| 23III | 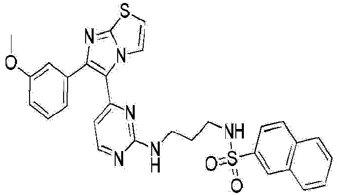 | 24III | 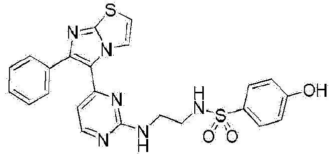 |
| 25III | 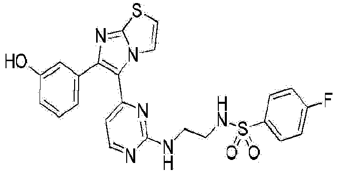 | 26III | 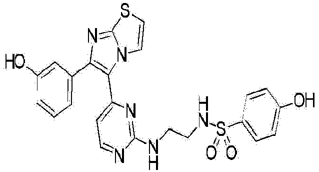 |
| 27III | 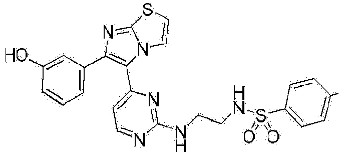 | 28III | 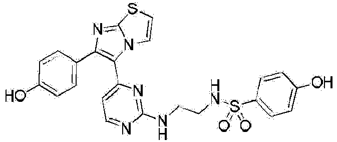 |
| 29III | 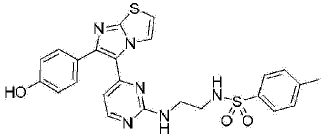 | 30III | 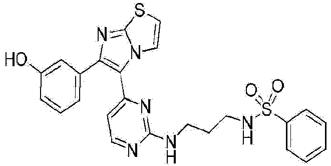 |
| 31III | 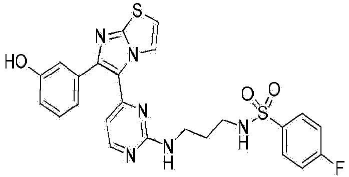 | 32III | 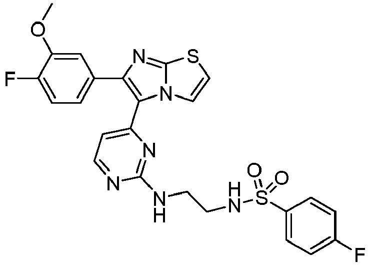 |
| 33III | 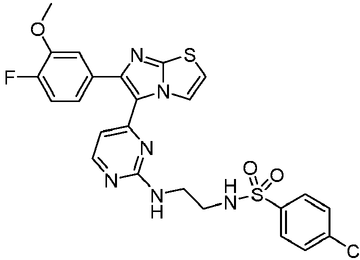 | 34III | 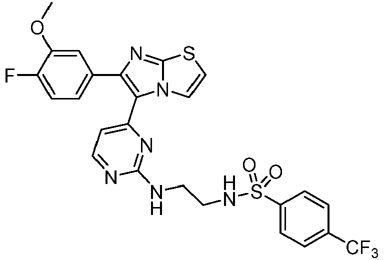 |
| 35III | 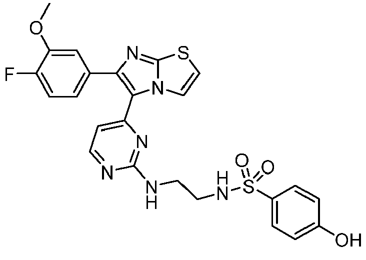 | 36III | 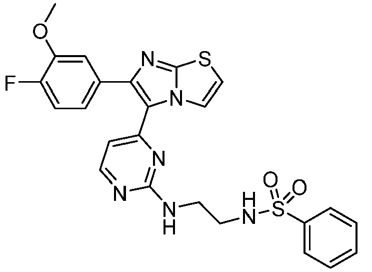 |
| 37III | 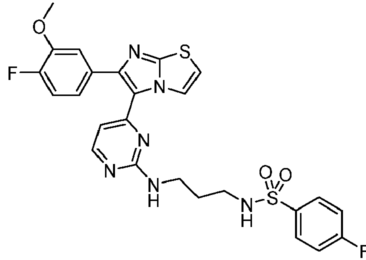 | 38III | 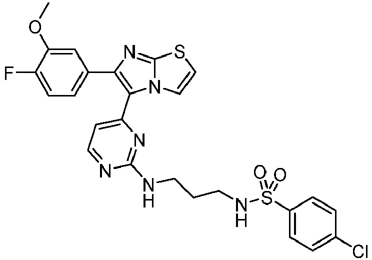 |
| 39III | 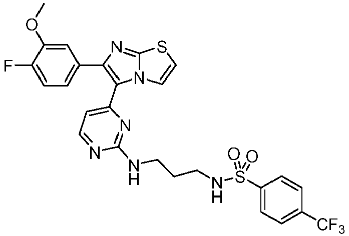 | 40III | 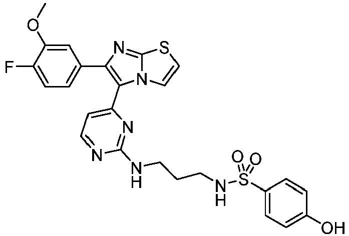 |
| 41III | 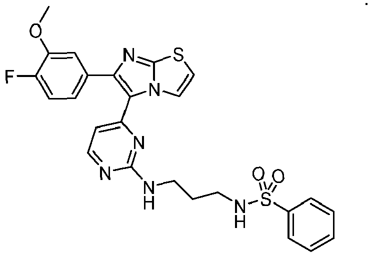 | 42III | 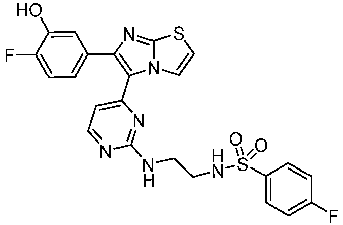 |
| 43III | 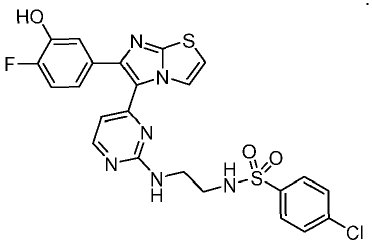 | 44III | 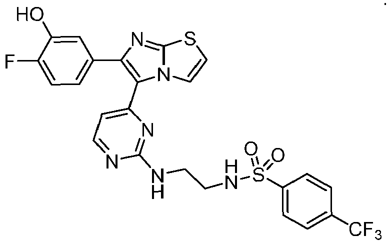 |
| 45III | 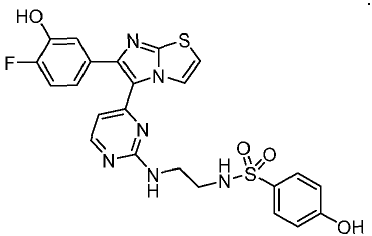 | 46III | 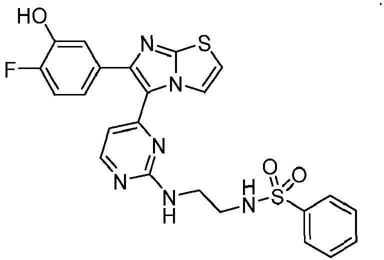 |
| 4IIII | 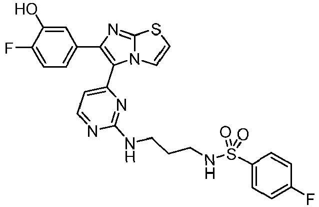 | 48III | 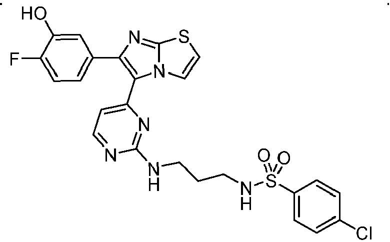 |
| 49III | 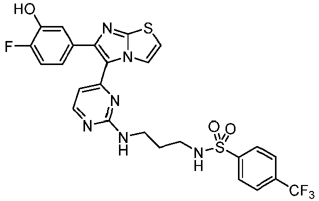 | 50III | 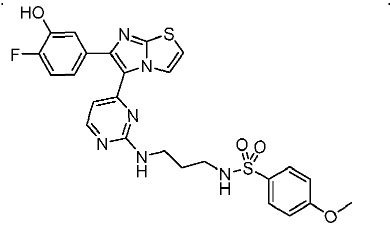 |
| 51III | 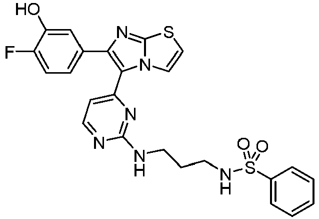 | 52III | 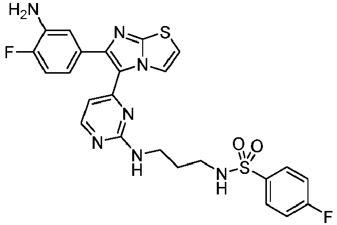 |
| 53III | 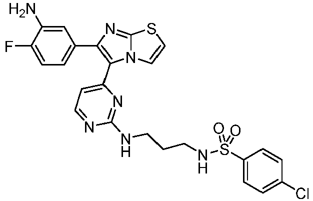 | 54III | 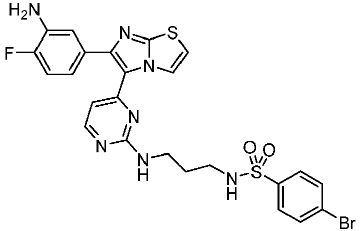 |
| 55III | 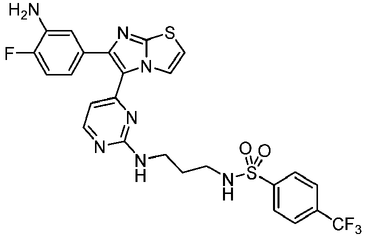 | 1IV | 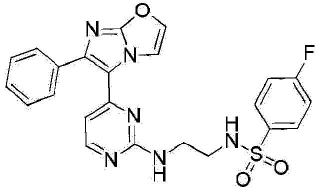 |
| 21V | 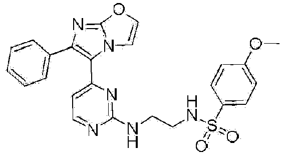 | 31V | 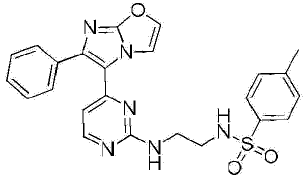 |
| 41V | 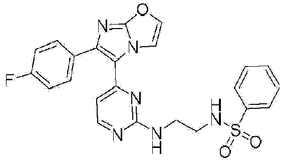 | 51V | 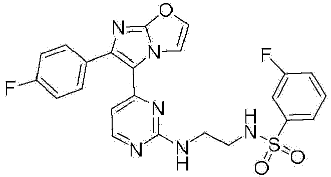 |
| 6IV | 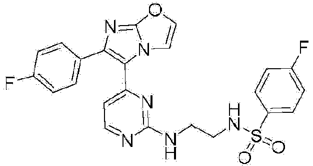 | 7IV | 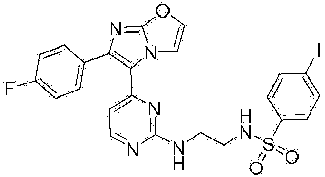 |
| 81V | 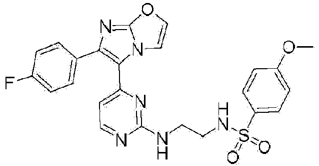 | 9IV | 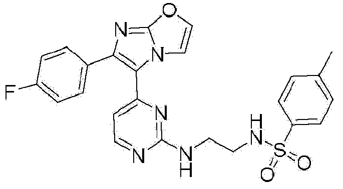 |
| 10IV | 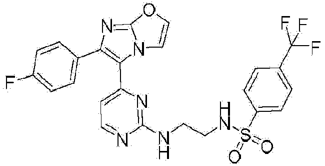 | 11IV | 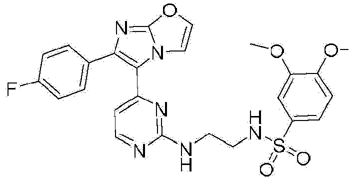 |
| 12IV | 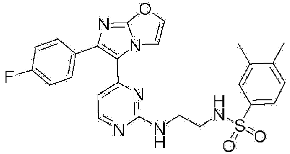 | 13IV | 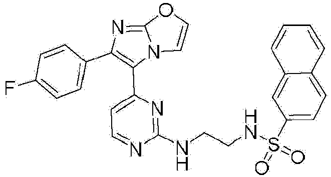 |
| 14IV | 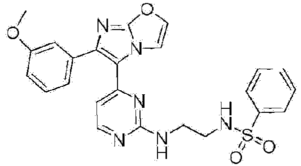 | 15IV | 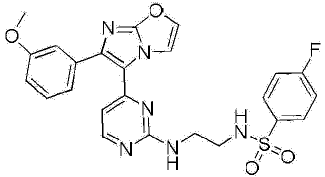 |
| 16IV | 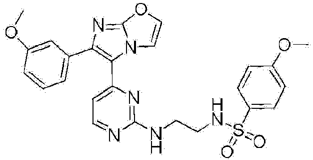 | 17IV | 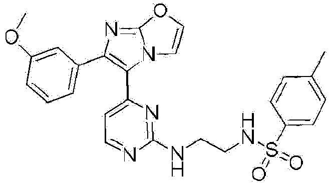 |
| 18IV | 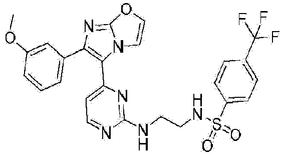 | 19IV | 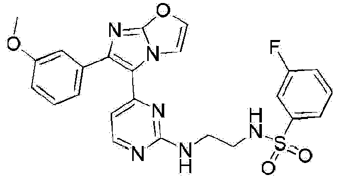 |
| 20IV | 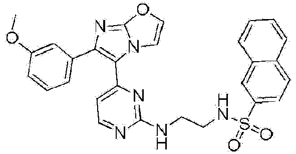 | 21IV | 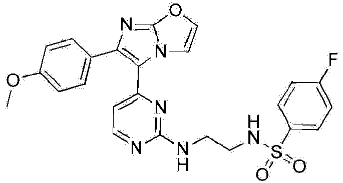 |
| 221V | 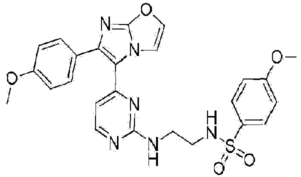 | 231V | 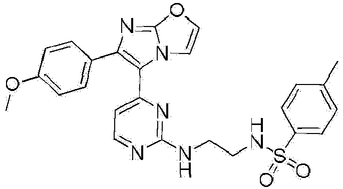 |
| 241V | 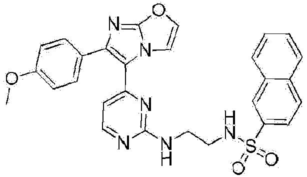 | 251V | 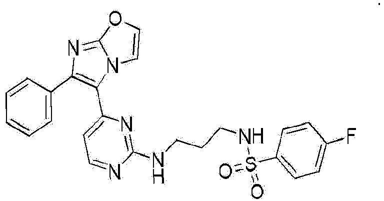 |
| 261V | 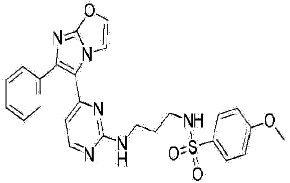 | 271V | 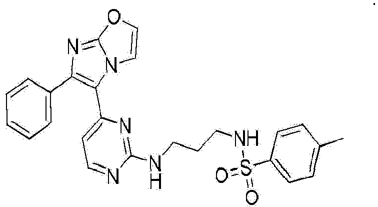 |
| 281V | 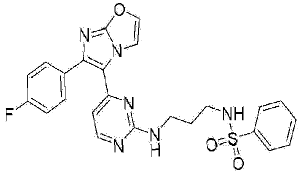 | 291V | 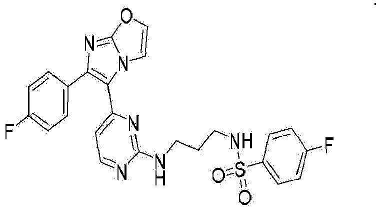 |
| 30IV | 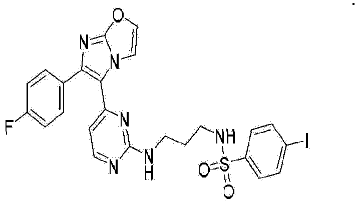 | 31IV | 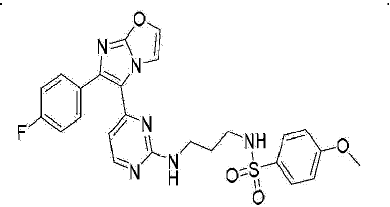 |
| 32IV | 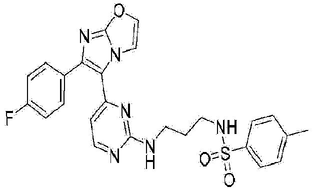 | 33IV | 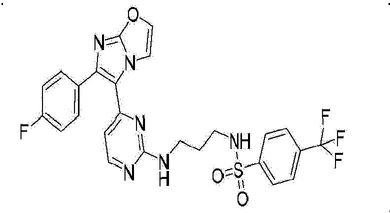 |
| 34IV | 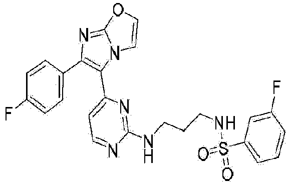 | 35IV | 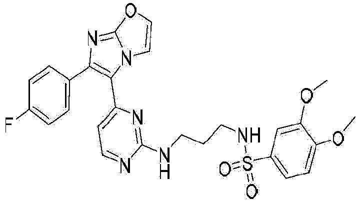 |
| 36IV | 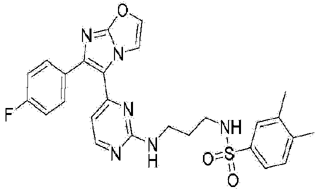 | 37IV | 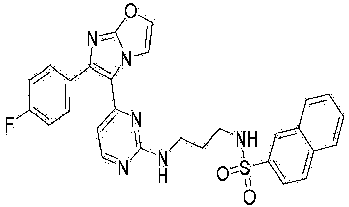 |
| 38IV | 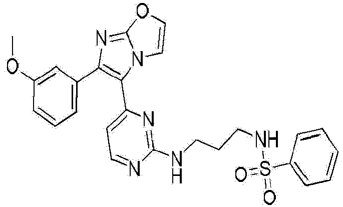 | 39IV | 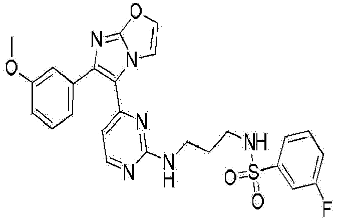 |
| 40IV | 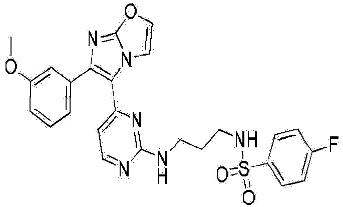 | 41IV | 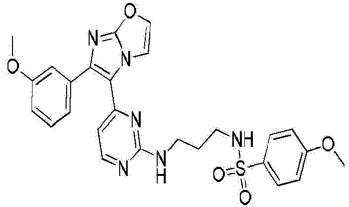 |
| 42IV | 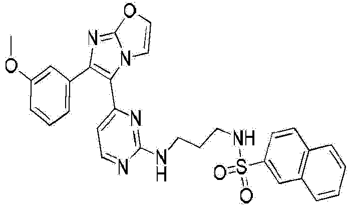 | 43IV | 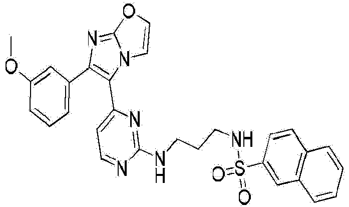 |
| 44IV | 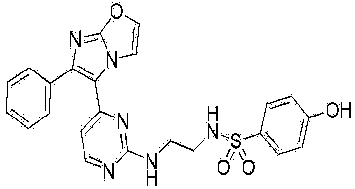 | 45IV | 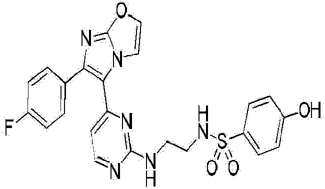 |
| 46IV | 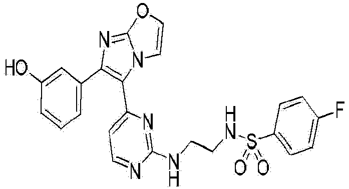 | 47IV | 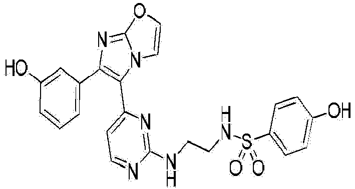 |
| 48IV | 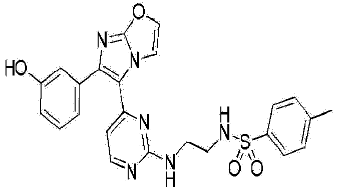 | 49IV | 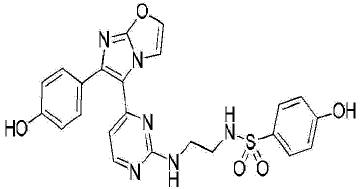 |
| 50IV | 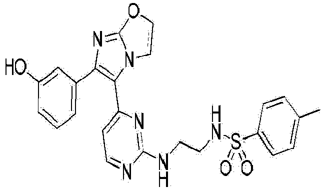 | 51IV | 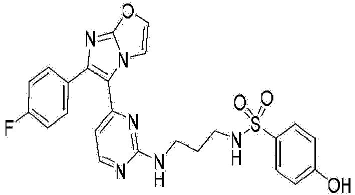 |
| 52IV | 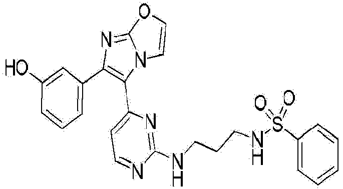 | 53IV | 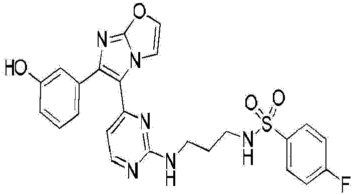 |
| 54IV | 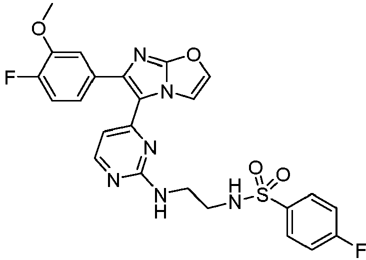 | 55IV | 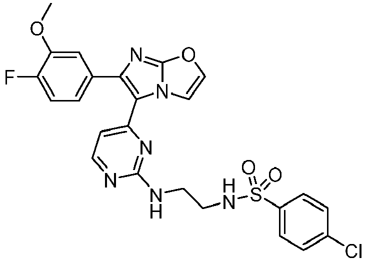 |
| 56IV | 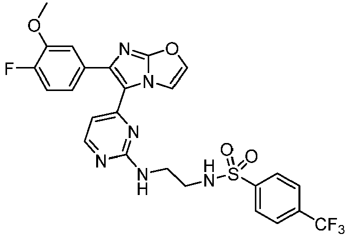 | 57IV | 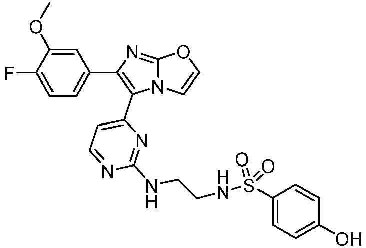 |
| 581V | 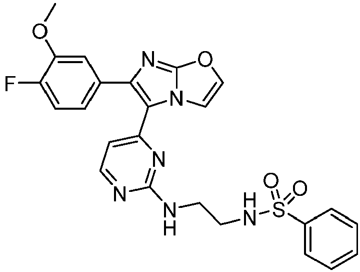 | 591V | 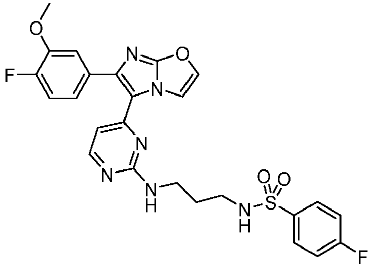 |
| 60IV | 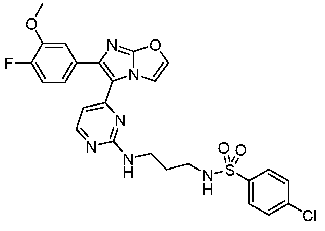 | 61IV | 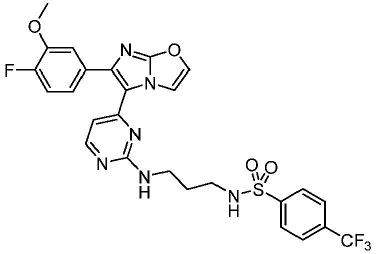 |
| 62IV | 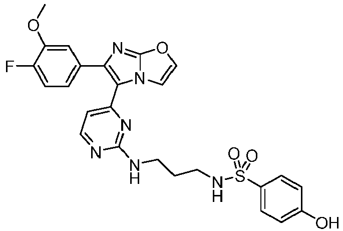 | 63IV | 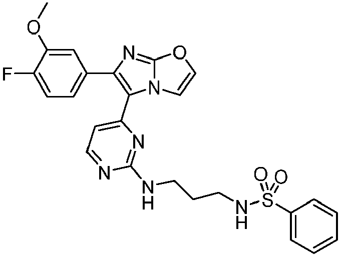 |
| 64IV | 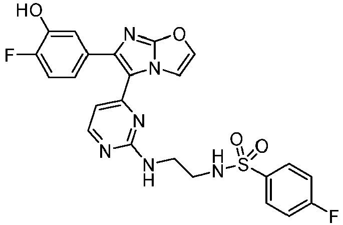 | 65IV | 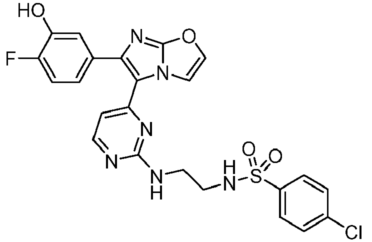 |
| 66IV | 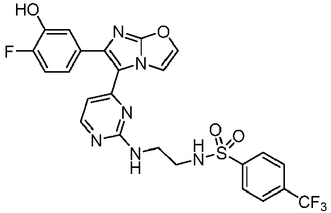 | 67IV | 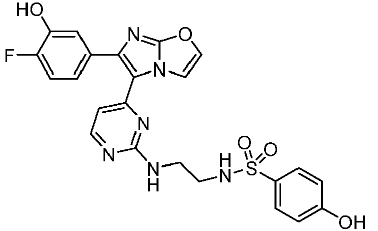 |
| 681V | 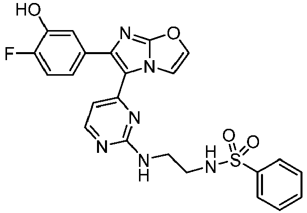 | 691V | 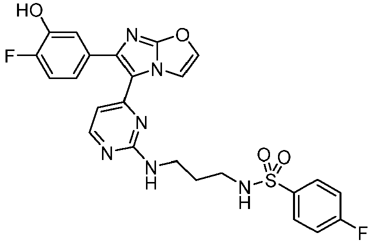 |
| 70IV | 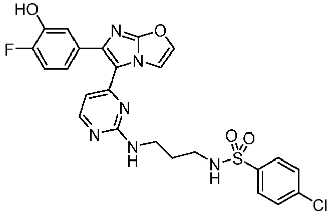 | 71IV | 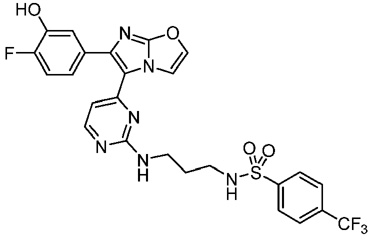 |
| 721V | 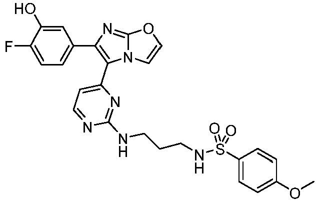 | 731V | 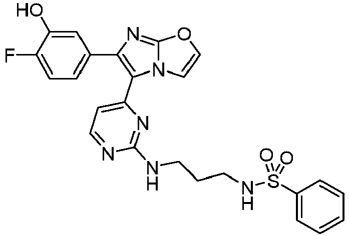 |

### <Experimental Example 1> Measurement of antiproliferative activity against melanoma cell lines

To measure inhibitory activities of imidazooxazole- or imidazothiazole-based compounds according to the present disclosure against proliferation of cancer cells at a cell level, the following experiments were carried out.

### (1) Screening of cancer cell line

According to the standard protocol (http://dtp.nci.nih.gov/dtpstandard/dwindex/index.jsp) of the US National Cancer Institute (NCI) (www.dtp.nci.nih.gov), screening of a cancer cell line panel was performed. Briefly, human cell lines were allowed to grow in an RPMI 1640 medium containing 5 % FBS and 2 mM L-glutamine. For a general screening experiment, cells were inoculated in 96-well microtiter plates in 100 µl at a plating density ranging from 5000 cells/well to 40,000 cells/well depending on the doubling time of individual cell lines. After cell inoculation, the microtiter plates were incubated at 37 °C, 5 % CO₂, 95 % air, and 100 % relative humidity for 24 hours prior to addition of test compounds. 24 hours later, two plates of each cell line were fixed *in situ* with trichloroacetic acid (TCA) to measure the cell population for each cell line at the time of test compound addition (Tz). Test compounds were solubilized in dimethyl sulfoxide at 400-fold the intended final maximum test concentration, and stored frozen prior to use. At the time of compound addition, an aliquot of frozen concentrate was thawed and diluted to twice the intended final maximum test concentration with a complete medium containing 50 µg/ml of gentamicin. Additional four concentration of 10-fold or 1/2 log serial dilutions were made to provide a total of five compound concentrations plus control. Aliquots of 100 µl of these different compound dilutions were added to appropriate microtiter wells already containing 100 µl of medium, resulting in the required final compound concentrations.

Following compound addition, the plates were incubated for additional 48 hours at 37 °C, 5 % CO₂, 95 % air, and 100 % relative humidity. For adherent cells, the assay was terminated by the addition of cold TCA. Cells were fixed *in situ* by the gentle addition of 50 µl of cold 50 % (w/v) TCA (final concentration, 10% TAC) and incubated at 4 °C for 60 minutes. The supernatant was discarded, and the plates were washed five times with tap water and air-dried. A sulforhodamine B (SRB) solution (100 µl) at 0.4 %(w/v) in 1 % acetic acid was added to each well, and plates were incubated at room temperature for 10 minutes. After staining, unbound dye was removed by washing five times with 1% acetic acid and the plates were air-dried. Bound stain was subsequently solubilized with 10 mM Trizma base, and the absorbance was read on an automated plate reader at a wavelength of 515 nm. For suspension cells, the methodology was the same except that the assay was terminated by fixing settled cells at the bottom of the wells by gently adding 50 µl of 80 % TCA (final concentration, 16 % TCA). Using the seven absorbance measurements [time zero (Tz), control growth (C), and test growth rate in the presence of compound at the five concentration levels (Ti)], the growth percentage was calculated at each of the compound concentration levels. Growth inhibition percentage was calculated as follows:
[(Ti-Tz)/(C-Tz)] × 100 for concentrations for which Ti>/=Tz
[(Ti-Tz)/Tz] × 100 for concentrations for which Ti<Tz.

Table 2. IC₅₀ values of main compounds against melanoma cell lines *in vitro* (none of these compounds are part of the invention).

**[Table 2]**

| | 1II | 2II | 5II | 6II | 8II | 2111 | 8111 | 25111 | 31III |
|---|---|---|---|---|---|---|---|---|---|
| A375 | 0.06 | 0.19 | 0.87 | 0.47 | 0.058 | ND | ND | 0.48 | 0.30 |
| LOX IMVI | ND | 2.53 | 2.30 | 4.36 | 2.15 | 3.94 | 3.26 | 4.11 | 0.56 |
| M14 | ND | 0.14 | 0.32 | 0.16 | 0.30 | 2.77 | 3.26 | 0.56 | 0.06 |
| MDA-MB-435 | ND | 0.37 | 0.74 | 0.49 | 0.38 | 1.22 | 3.26 | 0.93 | 0.24 |
| SK-MEL-2 | ND | 2.60 | 1.6 | 2.28 | 0.38 | 4.98 | 3.04 | 4.49 | 1.1 |
| SK-MEL-28 | ND | 0.13 | 0.22 | 0.24 | 0.17 | 5.79 | 3.68 | 0.66 | 0.05 |
| SK-MEL-5 | ND | 6.28 | 0.34 | 0.24 | 0.17 | 1.68 | 1.24 | 1.61 | 1.2 |
| UACC-62 | ND | 0.07 | 0.09 | 0.058 | 0.059 | 1.17 | 0.48 | 0.26 | 0.02 |

The test compounds showed proliferation-inhibitory activities against most of the melanoma cell lines shown in Table 2 (all 10 µM or less), indicating that the imidazooxazole- or imidazothiazole-based compounds according to the present disclosure have excellent inhibitory activities against melanoma.

### (2) Comparison of inhibitory activity against proliferation of A375P melanoma cell line

To examine whether the compounds (2II, 9II, 10II, and 1211 to 1511) of the present disclosure have remarkable effects, as compared with imidazothiazole-based compounds previously disclosed by the present inventors (M.S. Abdel-Maksoud et al./European Journal of Medicinal Chemistry 95 (2015) 453-463), inhibitory activities thereof against proliferation of A375P cell line which is a melanoma cell line were compared by the following experiments.

A375P cell line purchased from ATCC was cultured in a DMEM medium [containing 10% FBS and 1% penicillin/streptomycin] under 5% CO₂ at 37 °C. The cultured A375P cell line was collected using 0.05% trypsin-0.02% EDTA, and inoculated at a cell density of 5×10³ cells per well in a 96-well plate. To measure cell viability, MTT [3-(4,5-dimethylthiazol-2-yl)-2,5-diphenyltetrazolium bromide] assay (CellTiter 96 Assay, Promega) was performed as follows. MTT assay was performed in accordance with the guidelines of CellTiter 96^{®} (Promega). EnVision 2103 was used to read a value at a wavelength of 590 nm, and IC₅₀ was calculated using a software of GraphPad Prism 4.0.

Table 3. Inhibitory activities (IC₅₀ values (µM)) of main imidazothiazole-based compounds against proliferation of A375P cell line (not falling into the invention).

**[Table 3]**

| Compound | IC₅₀ (A375P) |
|---|---|
| 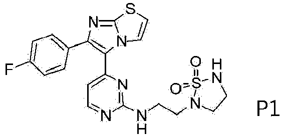 | 1.90 |
| 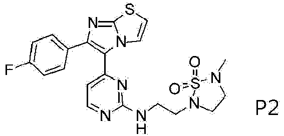 | 0.60 |
| 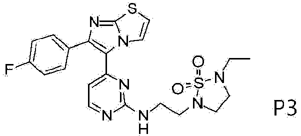 | 0.38 |
| 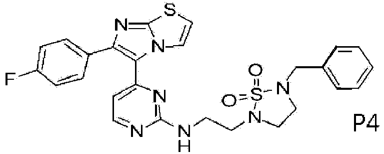 | >10 |
| 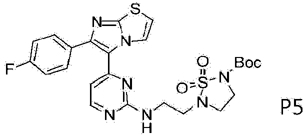 | >10 |
| 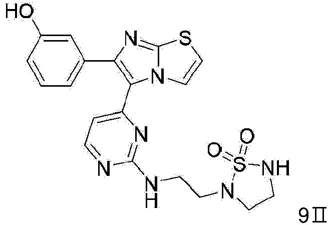 | 0.63 |
| 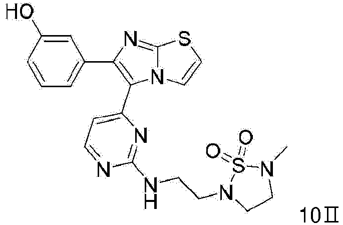 | 0.06 |
| 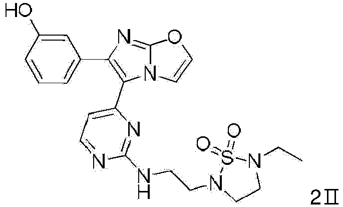 | 0.19 |
| 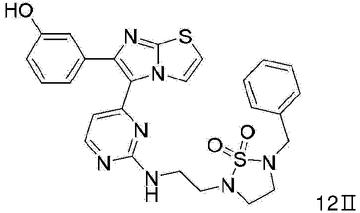 | 0.25 |
| 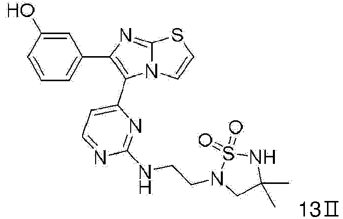 | 0.87 |
| 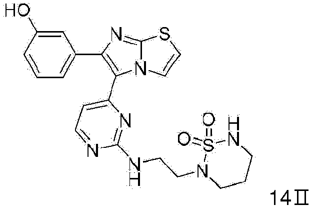 | 0.72 |
| 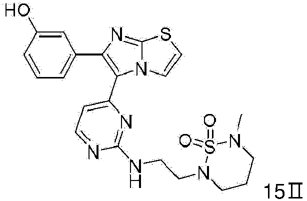 | 0.47 |
| 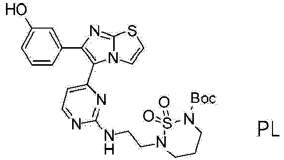 | 0.47 |

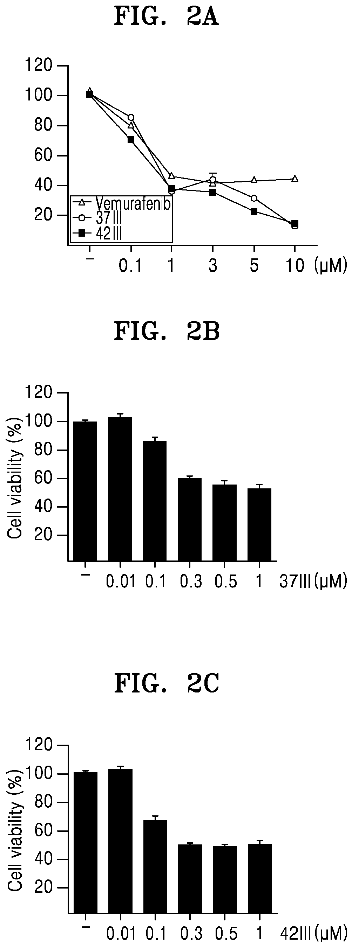

As shown in Table 3, most of the compounds 2II, 9II, 10II, and 12II to 15II of the present disclosure were found to have excellent proliferation-inhibitory effects, as compared with the known imidazothiazole-based compounds P1 to P5. When similar structures were compared with each other (compounds P1/9II, P2/10II, P3/2II, and P4/12II), it was more clearly found that the compounds of the present disclosure have more remarkable effects. Particularly, as shown in FIG. 2, when A of Chemical Formula P has two substituents R¹ and Y, it was shown that inhibitory activity against proliferation of the A375P melanoma cell line was excellent.

### <Experimental Example 2> Measurement of protein kinase enzymatic activity

Whether the imidazooxazole- and imidazothiazole-based compounds of the present disclosure inhibit kinase enzymatic activity involved with inhibitory activities against melanoma cell line proliferation was examined by the following method.

Reaction biology kinase hotspot service (http://www.reactionbiology.com) was used to measure IC₅₀. In a final reaction volume of 25 µL, kinase (5 mU to 10 mU) was incubated with 25 mM Tris (pH 7.5), 0.02 mM EGTA, 0.66 mg/ml of myelin basic protein, 10 mM magnesium acetate, and [³³P-ATP] (specific activity of about 500 CPM/pmol, concentration as required). The reaction was initiated by addition of Mg-ATP mix. After incubation for 40 min at room temperature, the reaction was stopped by addition of 5 µl of a 3% phosphoric acid solution. 10 pl_ of the reaction was then spotted onto a P30 filtermat and washed three times for 5 minutes in 75 mM phosphoric acid and once in methanol prior to drying and scintillation counting.

Table 4. Inhibitory effects against kinase activities of B-RAF, V600E-RAF, and C-RAF (nM)

**[Table 4]**

| | B-RAF | V600E-B-RAF | C-RAF |
|---|---|---|---|
| 1II (Reference) | 3.23 | 1.85 | 2.95 |
| 2II (Reference) | <0.04 | 0.67 | 0.75 |
| 2III (Reference) | 459 | 77 | ND |
| 8III (Reference) | 687 | 98 | ND |
| 25III (Reference) | 5.70 | 1.00 | ND |
| 3111 (Reference) | 4.19 | 0.98 | 7.83 |
| 37III | 8.8 nM | 1.66 nM | 13.8 nM |
| 38III | ND | 1.89 nM | ND |
| 42III | ND | 0.36 nM | ND |
| 43III | ND | 6.96 nM | ND |
| 46III | ND | 0.15 nM | ND |
| 48III | ND | 0.27 nM | ND |
| 50III | ND | 0.05 nM | ND |
| 52III | 2.68 nM | 1.03 nM | 5.41 nM |
| 60 IV | ND | 11.3 nM | ND |
| 64 IV | ND | 0.36 nM | ND |
| 65 IV | ND | 0.37 nM | ND |
| 70 IV | ND | 0.15 nM | ND |
| 72 IV | ND | 0.08 nM | ND |
| 73 IV | ND | 0.16 nM | ND |
| GW5074 | 8.52 | 2.96 | 2.58 |
| Vemurafenib | 100 | 31 | 48 |

As shown in Table 4, among the compounds according to the present disclosure, the compounds 1II, 2II, 25III, and 31III showed excellent inhibitory effects against enzymatic activities of B-RAF, V600E-RAF, and C-RAF, which were higher than those of GW5074 and vemurafenib. In particular, when A of formula P has two substituents R¹ and Y, it was found that inhibition of V600E-B-RAF enzyme activity was excellent.

Taken together, the compounds according to the present disclosure showed excellent inhibitory effects against protein kinases, in particular, many different protein kinases, for example, V600E RAF, B-RAF, C-RAF, MAPK14, FLT3, and GSK3β, each causing diseases associated with abnormal cell growth such as tumor cells, and thus the compounds may be usefully applied to prevention and treatment of tumor cell growth.

### <Experimental Example 3> Tumor-inhibitory effects in melanoma mouse model

To examine whether the inhibitory effects against proliferation of melanoma cells and the inhibitory effects against enzymatic activities of B-RAF, V600E-RAF, and C-RAF observed at the cell level are also reproduced in animal models, the following experiment was performed.

In this experiment, A375P cell line which is a human-derived malignant melanoma cell line was subcutaneously administered to nude mice to prepare xenograft models, and the test material (compound 211) was administered to the xenograft models to evaluate anti-cancer effects (G1: an excipient control group, G2: a group administered with 20 mg/kg/day of the test material, and G3: a group administered with 50 mg/kg/day of the test material).

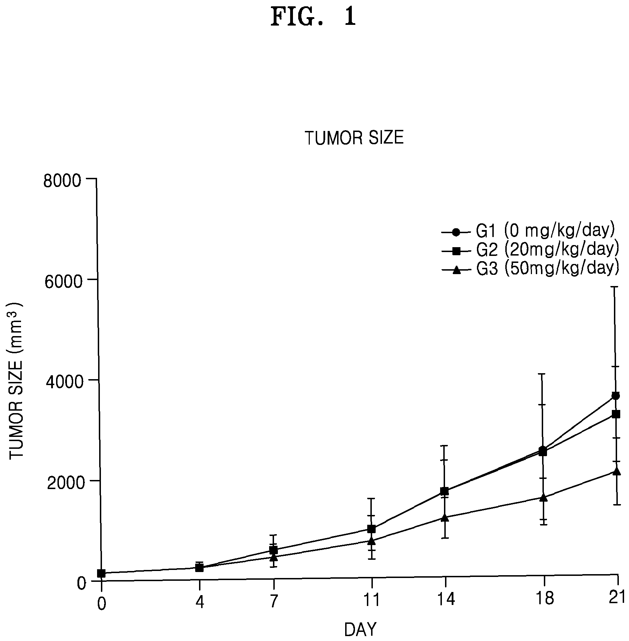

Results are shown in FIG. 1 and Table 5 below.

Table 5. Increase of tumor size in melanoma xenograft models (compound 2II) (not part of the invention).

**[Table 5]**

| Day | Group (mg/kg/day) | | |
|---|---|---|---|
| | G1 (0) | G2 (20 mg/kg/day) | G3 (50 mg/kg/day) |
| 0 | 148.94±6.23 | 145.08±20.85 | 149.36±20.91 |
| 4 | 271.05±85.51 | 271.88±60.05 | 276.08±86.17 |
| 7 | 552.89±311.28 | 556.42±161.21 | 442.88±73.08 |
| 11 | 984.73±589.89 | 978.91±258.24 | 754.16±211.86 |
| 14 | 1697.7±931.04 | 1711.5±631.79 | 1174.7+411.22 |
| 18 | 2512.6±1491.9 | 2438.5±962.99 | 1543.9±411.21 |
| 21 | 3557.1±2162.2 | 3200.6±944.99 | 2063.1±657.93 |

General symptoms were examined, and as a result, no deaths or no abnormalities in general symptoms were observed. Body weights were examined, and as a result, the body weight of the group (G3) administered with 50 mg/kg/day of the test material was significantly low at 14 days and 21 days after administration of the test material, as compared with that of the control group (G1), and the body weight gain of the group administered with 50 mg/kg/day of the test material was also significantly low, as compared with that of the excipient control group.

Tumor sizes were measured, and as a result, there was no significant difference between all experimental groups, but it was observed that increase of the tumor size was inhibited in a dose-dependent manner in the groups administered with the test material.

In conclusion, when intravenous administration of the test material to the malignant melanoma xenograft models prepared using nude mice was repeated twice per week for 3 weeks, increase of the tumor size was found to be inhibited in a dose-dependent manner, although the body weight of the test material-administered group was significantly low or tend to decrease and reduction in the body weight gain thereof was observed, as compared with that of the control group. Accordingly, it was confirmed that the compounds of the present disclosure show inhibitory activity against tumor growth in the animal test as well as at the cell level, suggesting that the compounds may have therapeutic and prophylactic effects on tumors.

## Claims

1. A compound represented by the following Chemical Formula P or a solvate, stereoisomer, or pharmaceutically acceptable salt thereof: 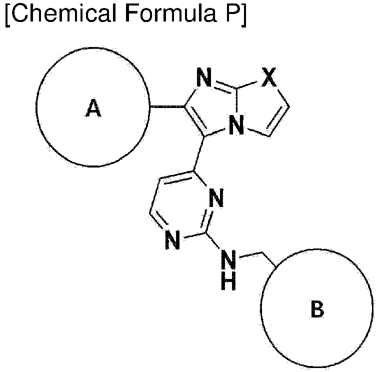 wherein, in Chemical Formula P,
X is O or S,
A is 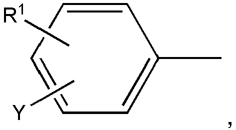 in which
R¹ is hydrogen, hydroxy, C₁-C₄ alkoxy, a halogen, nitro, or amino, and
Y is hydrogen, hydroxy, C₁-C₄ alkoxy, or a halogen;
B is 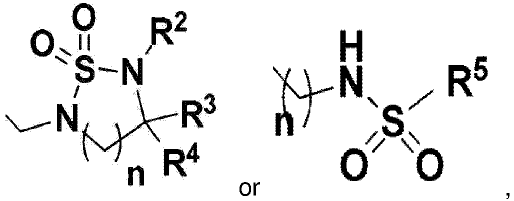 wherein
n = 1 or 2,
R², R³, and R⁴ are each independently hydrogen, *tert*-butyloxycarbonyl, or C₁-C₄ linear or branched alkyl, C₅-C₁₂ aryl, C₅-C₁₂ heteroaryl, or C₅-C₁₂ heterocycloalkyl unsubstituted or substituted with one or more substituents, and
R⁵ is M or M-Z, wherein M and Z are each independently C₅-C₁₂ aryl, C₅-C₁₂ heteroaryl, C₅-C₁₂ aryl C₁-C₄ alkyl, C₅-C₁₂ heteroaryl C₁-C₄ alkyl, C₅-C₁₂ cycloalkyl, or C₅-C₁₂ heterocycloalkyl unsubstituted or substituted with one or more substituents, wherein the one or more substituents are selected from the group consisting of halogen, C₁-C₄ linear or branched alkyl, C₁-C₄ alkoxy, trifluoromethyl, hydroxyl, amine, C₁-C₄ alkylamine, nitro, amide, C₁-C₄ alkylamide, urea, and acetyl,
wherein the compound represented by Chemical Formula P is selected from the group consisting of:
4-fluoro-N-(3-(4-(6-(4-fluoro-3-methoxyphenyl)imidazo[2,1-b]thiazol-5-yl) pyrimidin-2-ylamino)propyl)benzenesulfonamide;
4-chloro-N-(3-(4-(6-(4-fluoro-3-methoxyphenyl)imidazo[2,1-b]thiazol-5-yl) pyrimidin-2-ylamino)propyl)benzenesulfonamide;
4-fluoro-N-(2-(4-(6-(4-fluoro-3-hydroxyphenyl)imidazo[2,1-b]thiazol-5-yl) pyrimidin-2-ylamino)ethyl)benzenesulfonamide;
4-chloro-N-(2-(4-(6-(4-fluoro-3-hydroxyphenyl)imidazo[2,1-b]thiazol-5-yl) pyrimidin-2-ylamino)ethyl)benzenesulfonamide;
N-(2-(4-(6-(4-fluoro-3-hydroxyphenyl)imidazo[2,1-b]thiazol-5-yl)pyrimidin-2-yl amino)ethyl)benzenesulfonamide;
4-chloro-N-(3-(4-(6-(4-fluoro-3-hydroxyphenyl)imidazo[2,1-b]thiazol-5-yl) pyrimidin-2-ylamino)propyl)benzenesulfonamide;
N-(3-(4-(6-(4-fluoro-3-hydroxyphenyl)imidazo[2,1-b]thiazol-5-yl)pyrimidin-2-yl amino)propyl)-4-methoxybenzenesulfonamide;
N-(3-(4-(6-(3-amino-4-fluorophenyl)imidazo[2,1-b]thiazol-5-yl)pyrimidin-2-yl amino)propyl)-4-fluorobenzenesulfonamide;
4-chloro-N-(3-(4-(6-(4-fluoro-3-methoxyphenyl)imidazo[2,1-b]oxazol-5-yl) pyrimidin-2-ylamino)propyl)benzenesulfonamide;
4-fluoro-N-(2-(4-(6-(4-fluoro-3-hydroxyphenyl)imidazo[2,1-b]oxazol-5-yl) pyrimidin-2-ylamino)ethyl)benzenesulfonamide;
4-chloro-N-(2-(4-(6-(4-fluoro-3-hydroxyphenyl)imidazo[2,1-b]oxazol-5-yl) pyrimidin-2-ylamino)ethyl)benzenesulfonamide;
4-chloro-N-(3-(4-(6-(4-fluoro-3-hydroxyphenyl)imidazo[2,1-b]oxazol-5-yl) pyrimidin-2-ylamino)propyl)benzenesulfonamide;
N-(3-(4-(6-(4-fluoro-3-hydroxyphenyl)imidazo[2,1-b]oxazol-5-yl)pyrimidin-2-yl amino)propyl)-4-methoxybenzenesulfonamide; and
N-(3-(4-(6-(4-fluoro-3-hydroxyphenyl)imidazo[2,1-b]oxazol-5-yl)pyrimidin-2-yl amino)propyl)benzenesulfonamide.

2. A method of preparing the compound or the solvate, stereoisomer, or pharmaceutically acceptable salt thereof of claim 1, the method comprising:
cyclizing a compound of Chemical Formula 1 and a compound of Chemical Formula 2 to prepare a compound of Chemical Formula 3;
reacting the compound of Chemical Formula 3 with 4-chloro-2-(methylthio) pyrimidine to prepare a compound of Chemical Formula 4;
oxidizing the compound of Chemical Formula 4 to prepare a compound of Chemical Formula 5; and
reacting the compound of Chemical Formula 5 with an N-aminoethylcyclic sulfonamide derivative or an N-(2-aminoethyl)benzenesulfonamide derivative in the presence of a base to prepare the compound of Chemical Formula P.

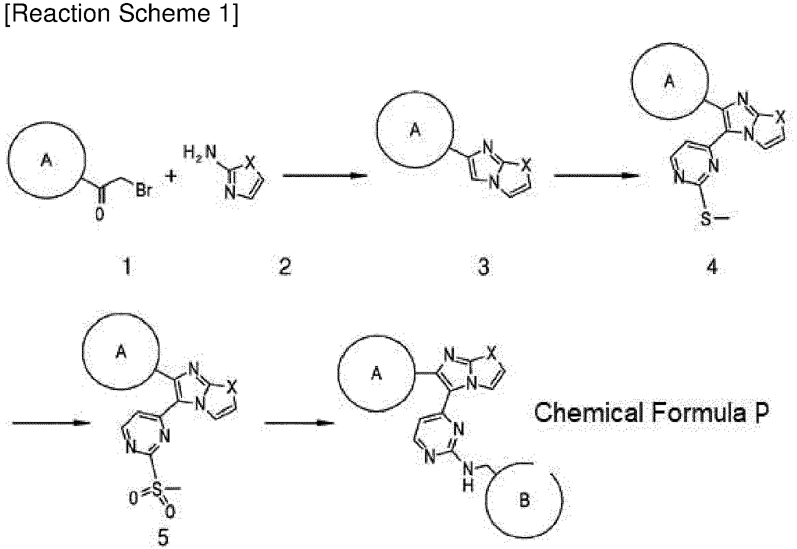

3. A pharmaceutical composition for use in preventing and treating tumors, the pharmaceutical composition comprising the compound or the solvate, stereoisomer, or pharmaceutically acceptable salt thereof of claim 1 as an active ingredient.

4. The pharmaceutical composition for use of claim 3, wherein the compound or the solvate, stereoisomer, or pharmaceutically acceptable salt thereof inhibits protein kinase to inhibit proliferation of tumor cells.

5. The pharmaceutical composition for use of claim 4, wherein the protein kinase is selected from the group consisting of V600E RAF, B-RAF, C-RAF, mitogen-activated protein kinase 14 (MAPK14), Fms-like tyrosine kinase 3 (FLT3), and glycogen synthase kinase 3 beta (GSK3β).

6. The pharmaceutical composition for use of claim 3, wherein the tumors are selected from the group consisting of lung cancer, liver cancer, esophageal cancer, stomach cancer, colorectal cancer, small intestine cancer, pancreatic cancer, melanoma, breast cancer, oral cancer, brain tumor, thyroid cancer, parathyroid cancer, renal cancer, cervical cancer, sarcoma, prostate cancer, urethral cancer, bladder cancer, testicular cancer, hematological malignancy, lymphoma, skin cancer, psoriasis, and fibroadenoma.

## Patentansprüche

1. Verbindung, die durch die folgende chemische Formel P dargestellt ist, oder ein Solvat, ein Stereoisomer oder ein pharmazeutisch unbedenkliches Salz davon: 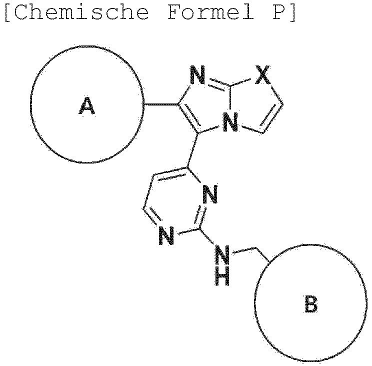 wobei in der chemischen Formel P
X O oder S ist,
A 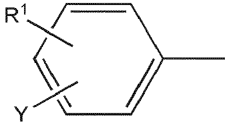 ist, wobei
R¹ Wasserstoff, Hydroxy, C₁-C₄-Alkoxy, ein Halogen, Nitro oder Amino ist, und
Y Wasserstoff, Hydroxy, C₁-C₄-Alkoxy oder ein Halogen ist;
B 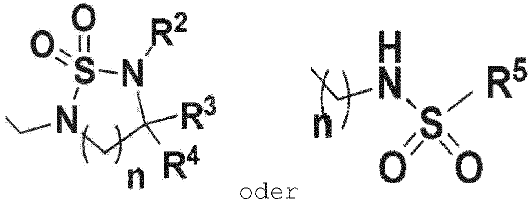 ist, wobei
n 1 oder 2 ist,
R², R³ und R⁴ jeweils unabhängig Wasserstoff, tert-Butyloxycarbonyl oder lineares oder verzweigtes C₁-C₄-Alkyl, C₅-C₁₂-Aryl, C₅-C₁₂-Heteroaryl oder C₅-C₁₂-Heterocycloalkyl sind, das unsubstituiert oder durch einen oder mehrere Substituenten substituiert ist, und
R⁵ M oder M-Z ist, wobei M und Z jeweils unabhängig C₅-C₁₂-Aryl, C₅-C₁₂-Heteroaryl, C₅-C₁₂-Aryl C₁-C₄-Alkyl, C₅-C₁₂-Heteroaryl-C₁-C₄-alkyl, C₅-C₁₂-Cycloalkyl oder C₅-C₁₂-Heterocycloalkyl sind, das unsubstituiert oder durch einen oder mehrere Substituenten substituiert ist, wobei der eine oder die mehreren Substituenten aus der Gruppe ausgewählt sind, die aus Halogen, linearem oder verzweigtem C₁-C₄-Alkoxy, C₁-C₄-Alkoxy, Trifluormethyl, Hydroxyl, Amin, C₁-C₄-Alkylamin, Nitro, Amid, C₁-C₄-Alkylamid, Harnstoff und Acetyl besteht,
wobei die Verbindung, die durch die chemische Formel P dargestellt ist, aus der Gruppe ausgewählt ist, die besteht aus:
4-Fluor-N-(3-(4-(6-(4-fluor-3-methoxyphenyl)imidazo[2,1-b ]thiazol-5-yl)pyrimidin-2-ylamino)propyl)benzolsulfonamid;
4-Chlor-N-(3-(4-(6-(4-fluor-3-methoxyphenyl)imidazo[2,1-b ]thiazol-5-yl)pyrimidin-2-ylamino)propyl)benzolsulfonamid;
4-Fluor-N-(2-(4-(6-(4-fluor-3-hydroxyphenyl)imidazo[2,1-b ]thiazol-5-yl)pyrimidin-2-ylamino)ethyl)benzolsulfonamid;
4-Chlor-N-(2-(4-(6-(4-fluor-3-hydroxyphenyl)imidazo[2,1-b ]thiazol-5-yl)pyrimidin-2-ylamino)ethyl)benzolsulfonamid;
N-(2-(4-(6-(4-Fluor-3-hydroxyphenyl)imidazo[2,1-b]thiazol -5-yl)pyrimidin-2-yl amino)ethyl)benzolsulfonamid;
4-Chlor-N-(3-(4-(6-(4-fluor-3-hydroxyphenyl)imidazo[2,1-b ]thiazol-5-yl)pyrimidin-2-ylamino)propyl)benzolsulfonamid;
N-(3-(4-(6-(4-Fluor-3-hydroxyphenyl)imidazo[2,1-b]thiazol -5-yl)pyrimidin-2-ylamino)propyl)-4-methoxybenzolsulfonamid;
N-(3-(4-(6-(3-Amino-4-fluorphenyl)imidazo[2,1-b]thiazol-5 -yl)pyrimidin-2-yl amino)propyl)-4-fluorbenzolsulfonamid;
4-Chlor-N-(3-(4-(6-(4-fluor-3-methoxyphenyl)imidazo[2,1-b ]oxazol-5-yl)pyrimidin-2-ylamino)propyl)benzolsulfonamid;
4-Fluor-N-(2-(4-(6-(4-fluor-3-hydroxyphenyl)imidazo[2,1-b ]oxazol-5-yl)pyrimidin-2-ylamino)ethyl)benzolsulfonamid;
4-Chlor-N-(2-(4-(6-(4-fluor-3-hydroxyphenyl)imidazo[2,1-b ]oxazol-5-yl)pyrimidin-2-ylamino)ethyl)benzolsulfonamid;
4-Chlor-N-(3-(4-(6-(4-fluor-3-hydroxyphenyl)imidazo[2,1-b ]oxazol-5-yl)pyrimidin-2-ylamino)propyl)benzolsulfonamid;
N-(3-(4-(6-(4-Fluor-3-hydroxyphenyl)imidazo[2,1-b]oxazol-5-yl)pyrimidin-2-ylamino)propyl)-4-methoxybenzolsulfonamid; und
N-(3-(4-(6-(4-Fluor-3-hydroxyphenyl)imidazo[2,1-b]oxazol-5-yl)pyrimidin-2-ylamino)propyl)benzolsulfonamide.

2. Verfahren zum Herstellen der Verbindung oder des Solvats, des Stereoisomers oder des pharmazeutisch unbedenklichen Salzes davon nach Anspruch 1, wobei das Verfahren umfasst:
Cyclisieren einer Verbindung der chemischen Formel 1 und einer Verbindung der chemischen Formel 2, um eine Verbindung der chemischen Formel 3 herzustellen;
Umsetzen der Verbindung der chemischen Formel 3 mit 4-Chlor-2-(methylthio)pyrimidin, um eine Verbindung der chemischen Formel 4 herzustellen;
Oxidieren der Verbindung der chemischen Formel 4, um eine Verbindung der chemischen Formel 5 herzustellen; und
Umsetzen der Verbindung der chemischen Formel 5 mit einem Derivat von cyclischem N-aminoethylsulfonamid oder einem N-(2-Aminoethyl)benzolsulfonamidderivat in Gegenwart einer Base, um die Verbindung der chemischen Formel P herzustellen.

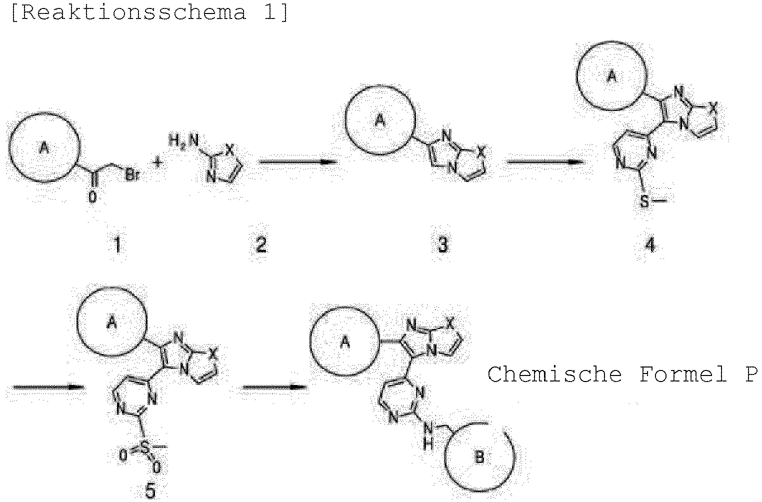

3. Pharmazeutische Zusammensetzung zur Verwendung bei der Prävention und Behandlung von Tumoren, wobei die pharmazeutische Zusammensetzung die Verbindung oder das Solvat, das Stereoisomer oder das pharmazeutisch unbedenkliche Salz davon nach Anspruch 1 als Wirkstoff umfasst.

4. Pharmazeutische Zusammensetzung zur Verwendung nach Anspruch 3, wobei die Verbindung oder das Solvat, das Stereoisomer oder das pharmazeutisch unbedenkliche Salz davon Proteinkinase hemmt, um die Proliferation von Tumorzellen zu hemmen.

5. Pharmazeutische Zusammensetzung zur Verwendung nach Anspruch 4, wobei die Proteinkinase aus der Gruppe ausgewählt ist, die aus V600E RAF, B-RAF, C-RAF, mitogenaktivierter Proteinkinase 14 (MAPK14), Fms-ähnlicher Tyrosinkinase 3 (FLT3) und Glycogensynthasekinase 3 beta (GSK3β) besteht.

6. Pharmazeutische Zusammensetzung zur Verwendung nach Anspruch 3, wobei die Tumoren aus der Gruppe ausgewählt sind, die aus Lungenkrebs, Leberkrebs, Speiseröhrenkrebs, Magenkrebs, Kolorektalkrebs, Dünndarmkrebs, Bauchspeicheldrüsenkrebs, Melanom, Brustkrebs, Mundkrebs, Hirntumor, Schilddrüsenkrebs, Nebenschilddrüsenkrebs, Nierenkrebs, Gebärmutterhalskrebs, Sarkom, Prostatakrebs, Harnröhrenkrebs, Blasenkrebs, Hodenkrebs, hämatologischer Malignität, Lymphom, Hautkrebs, Psoriasis und Fibroadenom besteht.

## Revendications

1. Composé représenté par la Formule Chimique P suivante ou un solvate, un stéréoisomère ou un sel pharmaceutiquement acceptable de celui-ci : 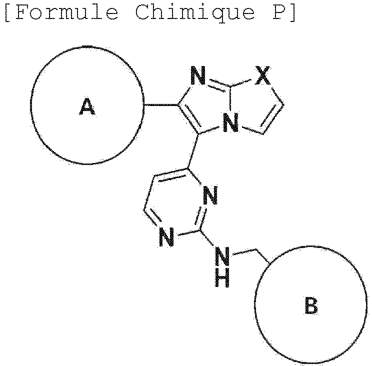 dans lequel, dans la Formule Chimique P,
X est O ou S,
A est 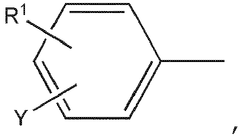 dans lequel
R¹ est un hydrogène, un hydroxy, un alcoxy en C₁-C₄, un halogène, un nitro ou un amino, et
Y est un hydrogène, un hydroxy, un alcoxy en C₁-C₄ ou un halogène ;
B est 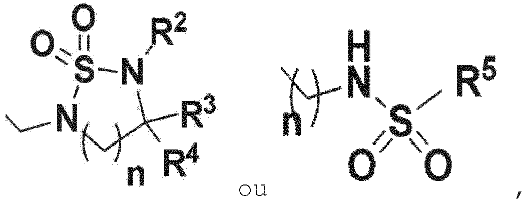 dans lequel
n = 1 ou 2,
R², R³ et R⁴ sont chacun indépendamment un hydrogène, un *tert-*butyloxycarbonyle ou un alkyle en C₁-C₄ linéaire ou ramifié, un aryle en C₅-C₁₂, un hétéroaryle en C₅-C₁₂ ou un hétérocycloalkyle en C₅-C₁₂ non substitué ou substitué par un ou plusieurs substituants, et
R⁵ est M ou M-Z, dans lequel M et Z sont chacun indépendamment un aryle en C₅-C₁₂, un hétéroaryle en C₅-C₁₂, un (aryl en C₅-C₁₂)-alkyle en C₁-C₄, un (hétéroaryl en C₅-C₁₂)-alkyle en C₁-C₄, un cycloalkyle en C₅-C₁₂ ou un hétérocycloalkyle en C₅-C₁₂ non substitué ou substitué par un ou plusieurs substituants, dans lequel les un ou plusieurs substituants sont choisis dans le groupe consistant en un halogène, un alkyle en C₁-C₄ linéaire ou ramifié, un alcoxy en C₁-C₄, un trifluorométhyle, un hydroxyle, une amine, une alkylamine en C₁-C₄, un nitro, un amide, un alkylamide en C₁-C₄, une urée et un acétyle,
dans lequel le composé représenté par la Formule Chimique P est choisi dans le groupe consistant en :
le 4-fluoro-N-(3-(4-(6-(4-fluoro-3-méthoxyphényl)imidazo[2,1-b]thi azol-5-yl)pyrimidin-2-ylamino)propyl)benzènesulfonamide ;
le 4-chloro-N-(3-(4-(6-(4-fluoro-3-méthoxyphényl)imidazo[2,1-b]thi azol-5-yl)pyrimidin-2-ylamino)propyl)benzènesulfonamide ;
le 4-fluoro-N-(2-(4-(6-(4-fluoro-3-hydroxyphényl)imidazo[2,1-b]thi azol-5-yl)pyrimidin-2-ylamino)éthyl)benzènesulfonamide ;
le 4-chloro-N-(2-(4-(6-(4-fluoro-3-hydroxyphényl)imidazo[2,1-b]thi azol-5-yl)pyrimidin-2-ylamino)éthyl)benzènesulfonamide ;
le N-(2-(4-(6-(4-fluoro-3-hydroxyphényl)imidazo[2,1-b]thiazol-5-yl )pyrimidin-2-ylamino)éthyl)benzènesulfonamide ;
le 4-chloro-N-(3-(4-(6-(4-fluoro-3-hydroxyphényl)imidazo[2,1-b]thi azol-5-yl)pyrimidin-2-ylamino)propyl)benzènesulfonamide ;
le N-(3-(4-(6-(4-fluoro-3-hydroxyphényl)imidazo[2,1-b]thiazol-5-yl )pyrimidin-2-ylamino)propyl)-4-méthoxybenzènesulfonamide ;
le N-(3-(4-(6-(3-amino-4-fluorophényl)imidazo[2,1-b]thiazol-5-yl)p yrimidin-2-ylamino)propyl)-4-fluorobenzènesulfonamide ;
4-chloro-N-(3-(4-(6-(4-fluoro-3-méthoxyphényl)imidazo[2,1-b]oxazol-5-yl)pyrimidin-2-ylamino)propyl)benzènesulfonamide ;
le 4-fluoro-N-(2-(4-(6-(4-fluoro-3-hydroxyphényl)imidazo[2,1-b]oxa zol-5-yl)pyrimidin-2-ylamino)éthyl)benzènesulfonamide ;
le 4-chloro-N-(2-(4-(6-(4-fluoro-3-hydroxyphényl)imidazo[2,1-b]oxa zol-5-yl)pyrimidin-2-ylamino)éthyl)benzènesulfonamide ;
le 4-chloro-N-(3-(4-(6-(4-fluoro-3-hydroxyphényl)imidazo[2,1-b]oxa zol-5-yl)pyrimidin-2-ylamino)propyl)benzènesulfonamide ;
le N-(3-(4-(6-(4-fluoro-3-hydroxyphényl)imidazo[2,1-b]oxazol-5-yl) pyrimidin-2-ylamino)propyl)-4-méthoxybenzènesulfonamide ; et
le N-(3-(4-(6-(4-fluoro-3-hydroxyphényl)imidazo[2,1-b]oxazol-5-yl) pyrimidin-2-ylamino)propyl)benzènesulfonamide.

2. Procédé de préparation du composé ou du solvate, stéréoisomère ou sel pharmaceutiquement acceptable de celui-ci selon la revendication 1, le procédé comprenant :
la cyclisation d'un composé de Formule Chimique 1 et d'un composé de Formule Chimique 2 pour préparer un composé de Formule Chimique 3 ;
la mise en réaction du composé de Formule Chimique 3 avec de la 4-chloro-2-(méthylthio)pyrimidine pour préparer un composé de Formule Chimique 4 ;
l'oxydation du composé de Formule Chimique 4 pour préparer un composé de Formule Chimique 5 ; et
la mise en réaction du composé de Formule Chimique 5 avec un dérivé de N-aminoéthylsulfonamide cyclique ou un dérivé de N-(2-aminoéthyl)benzènesulfonamide en présence d'une base pour préparer le composé de Formule Chimique P.

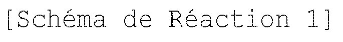

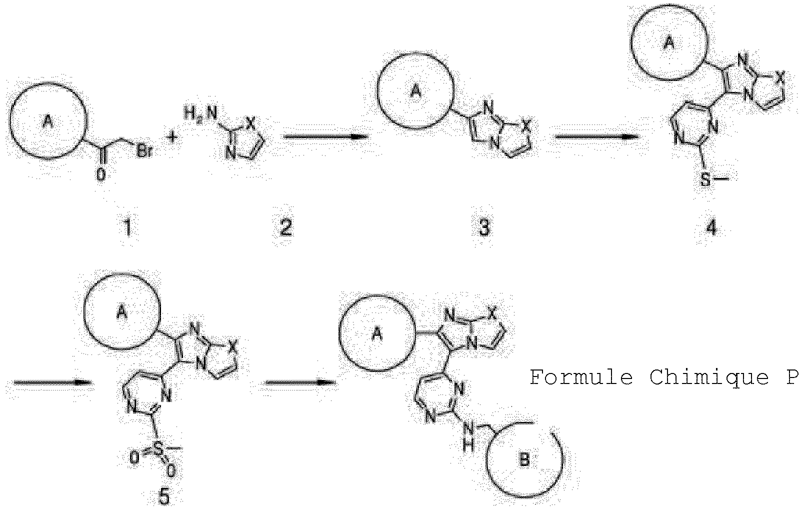

3. Composition pharmaceutique pour une utilisation dans la prévention et le traitement de tumeurs, la composition pharmaceutique comprenant le composé ou le solvate, stéréoisomère ou sel pharmaceutiquement acceptable de celui-ci selon la revendication 1 en tant qu'ingrédient actif.

4. Composition pharmaceutique pour une utilisation selon la revendication 3, dans laquelle le composé ou le solvate, stéréoisomère ou sel pharmaceutiquement acceptable de celui-ci inhibe une protéine kinase pour inhiber la prolifération de cellules tumorales.

5. Composition pharmaceutique pour une utilisation selon la revendication 4, dans laquelle la protéine kinase est choisie dans le groupe consistant en V600E RAF, B-RAF, C-RAF, la protéine kinase 14 activée par un à)mitogène (MAPK14), la tyrosine kinase 3 de type Fms (FLT3) et la glycogène synthase kinase 3 bêta (GSK3β).

6. Composition pharmaceutique pour une utilisation selon la revendication 3, dans laquelle les tumeurs sont choisies dans le groupe consistant en le cancer du poumon, le cancer du foie, le cancer de l'œsophage, le cancer de l'estomac, le cancer colorectal, le cancer de l'intestin grêle, le cancer du pancréas, le mélanome, le cancer du sein, le cancer de la bouche, la tumeur cérébrale, le cancer de la thyroïde, le cancer de la parathyroïde, le cancer du rein, le cancer du col de l'utérus, le sarcome, le cancer de la prostate, le cancer de l'urètre, le cancer de la vessie, le cancer des testicules, l'hémopathie maligne, le lymphome, le cancer de la peau, le psoriasis et le fibroadénome.
